# EUROPEAN PATENT SPECIFICATION

(11) **EP 1 395 604 B1**
(45) Date of publication and mention of the grant of the patent: **25.06.2008**
(21) Application number: 02730498.9
(22) Date of filing: 11.06.2002
(51) Int. Cl.: C07J 31/00, C07J 17/00, C07J 33/00, C07J 43/00, A61K 31/58, A61P 5/44

(54) **NOVEL ANTI INFLAMMATORY 17.ALPHA.-HETEROCYCLIC-ESTERS OF 17.BETA.-CARBOTHIOATE ANDROSTANE DERIVATIVES**
NEUE ANTI INFLAMMATORISCHE 17.ALPHA.-HETEROZYKLISCHE ESTER VON 17.BETA.-CARBOTHIOAT ANDROSTAN DERIVATIVEN
17 ALPHA ESTERS HETEROCYCLIQUES ANTI-INFLAMMATOIRES DE DERIVES DE 17 BETA CARBOTHIOATE ANDROSTANE

(30) Priority: 12.06.2001 GB 0114338; 05.02.2002 GB 0202636
(43) Date of publication of application: 10.03.2004
(73) Proprietor: GLAXO GROUP LIMITED, Greenford, Middlesex UB6 0NN (GB)
(72) Inventor: BIGGADIKE, Keith GlaxoSmithKline, Hertfordshire SG1 2NY (GB); JONES, Paul GlaxoSmithKline, Hertfordshire SG1 2NY (GB)
(74) Representative: Pritchard, Judith
(86) International application number: PCT/GB2002/002686
(87) International publication number: WO 2002/100879

(56) References cited:
- EP-A- 0 057 401
- WO-A-02/00679
- WO-A-02/12265
- WO-A-02/12266
- US-A- 4 188 385
- US-A- 4 263 289
- US-A- 4 335 121
- PHILLIPPS G H ET AL: "SYNTHESIS AND STRUCTURE-ACTIVITY RELATIONSHIPS IN A SERIES OF ANTIINFLAMMATORY CORTICOSTEROID ANALOGUES, HALOMETHYL ANDROSTANE- 17BETA-CARBOTHIOATES AND-17BETA-CARBOSELENOATES" JOURNAL OF MEDICINAL CHEMISTRY, AMERICAN CHEMICAL SOCIETY. WASHINGTON, US, vol. 37, no. 22, 1 October 1994 (1994-10-01), pages 3717-3729, XP002025925 ISSN: 0022-2623
- ISOGAI M ET AL: "BINDING AFFINITIES OF MOMETASONE FUROATE AND RELATED COMPOUNDS INCLUDING ITS METABOLITES FOR THE GLUCOCORTICOID RECEPTOR OF RAT SKIN TISSUE" JOURNAL OF STEROID BIOCHEMISTRY AND MOLECULAR BIOLOGY, ELSEVIER SCIENCE LTD., OXFORD, GB, vol. 44, no. 2, 1993, pages 141-145, XP001040943 ISSN: 0960-0760
- SHAPIRO E L ET AL: "17 HETEROAROYL ESTERS OF CORTICOSTEROIDS 2. 11-BETA HYDROXY SERIES" JOURNAL OF MEDICINAL CHEMISTRY, AMERICAN CHEMICAL SOCIETY. WASHINGTON, US, vol. 30, no. 9, 1987, pages 1581-1588, XP002153839 ISSN: 0022-2623
- POPPER T L ET AL: "STRUCTURE-ACTIVITY RELATIONSHIP OF A SERIES OF NOVEL TOPICAL CORTICOSTEROIDS" JOURNAL OF STEROID BIOCHEMISTRY, PERGAMON PRESS PLC, GB, vol. 27, no. 4 - 6, 1987, pages 837-843, XP000972215 ISSN: 0022-4731
- KNOBIL K ET AL: "ADDING SALMETEROL IS MORE EFFECTIVE THAN INCREASING THE DOSE OF FLUTICASONE FOR PATIENTS WITH ASTHMA WHO ARE SYMPTOMATIC ON LOW DOSE FLUTICASONE" EUROPEAN RESPIRATORY REVIEW, COPENHAGEN, DK, vol. 12, no. SUPPL 29, December 1998 (1998-12), pages 19S-20S, XP000992769

## Description

The present invention relates to novel anti-inflammatory and anti-allergic compounds of the androstane series and to processes for their preparation. The present invention also relates to pharmaceutical formulations containing the compounds and to therapeutic uses thereof, particularly for the treatment of inflammatory and allergic conditions. ,

Glucocorticosteroids which have anti-inflammatory properties are known and are widely used for the treatment of inflammatory disorders or diseases such as asthma and rhinitis. For example, US Patent 4335121 discloses 6α, 9α-Difluoro-17α-(1-oxopropoxy)-11β-hydroxy-16α-methyl-3-oxo-androsta-1,4-diene-17β-carbothioic acid S-fluoromethyl ester (known by the generic name of fluticasone propionate) and derivatives thereof. The use of glucocorticoids generally, and especially in children, has been limited in some quarters by concerns over potential side effects. The side effects that are feared with glucocorticoids include suppression of the Hypothalamic-Pituitary-Adrenal (HPA) axis, effects on bone growth in children and on bone density in the elderly, ocular complications (cataract formation and glaucoma) and skin atrophy. Certain glucocorticoid compounds also have complex paths of metabolism wherein the production of active metabolites may make the pharmacodynamics and pharmacokinetics of such compounds difficult to understand. Whilst the modern steroids are very much safer than those originally introduced it remains an object of research to produce new molecules which have excellent anti-inflammatory properties, with predictable pharmacokinetic and pharmacodynamic properties, with an attractive side effect profile, and with a convenient treatment regime.

Certain novel androstane derivatives are disclosed in WO02/12265, WO02/12266 (Glaxo Group) and WO02/00679 (Novartis), these three documents being published after the earliest priority date of this patent application.

We have identified a novel series of glucocorticoids, which substantially meets these objectives.

Thus, according to one aspect of the invention, there is provided a compound of formula (I) 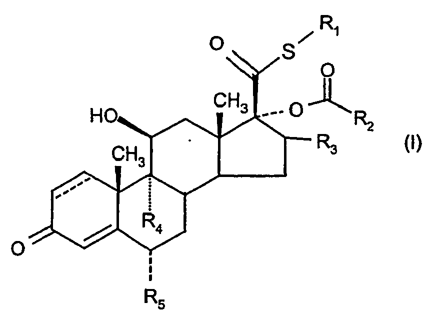 wherein
R₁ represents C₁₋₆ alkyl or C₁₋₆ haloalkyl;
R₂ represents a 4-7 membered non-aromatic ring containing 1-3 heteroatoms selected from O, N and S optionally substituted with one or more methyl, ethyl or halogen groups;
R₃ represents hydrogen, methyl (which may be in either the α or β configuration) or methylene;
R₄ and R₅ are the same or different and each represents hydrogen or halogen; and
-̅-̅-̅-̅-̅ represents a single or a double bond;
and salts and solvates thereof.

Examples of salts of compounds of formula (I) include physiologically acceptable salts which may be formed with basic compounds (such as when R₂ contains a secondary nitrogen atom) eg. acetate, benzoate, citrate, succinate, lactate, tartrate, fumarate and maleate.

Examples of solvates include hydrates.

References hereinafter to a compound according to the invention includes both compounds of formula (I) and salts and solvates thereof, particularly pharmaceutically acceptable salts and solvates.

We prefer to employ the compound of formula (I) in the form of a single diastereoisomer.

Examples of C₁₋₆ haloalkyl that R₁ may represent include C₁₋₆ alkyl substituted by 1-3 halogen atoms, preferably 1 halogen atom. Preferred halogen atoms are selected from bromine, chlorine and fluorine.

We prefer R₁ to represent fluoromethyl, chloromethyl, bromomethyl or 2'-fluoroethyl, especially fluoromethyl.

Non-aromatic rings for R₂ include rings that are saturated or partially unsaturated (eg. containing one double bond). Preferably, R₂ will be a saturated ring. We prefer that R₂ contains 1 or 2 heteroatoms, especially 1 heteroatom. We prefer that the heteroatom(s) are selected from 0 and S. We prefer R₂ to be a 4-6 membered ring, especially a 4 or 5 membered ring, particularly a 5-membered ring.

Example R₂ groups include tetrahydrofuranyl (eg tetrahydrofuran-2-yl and tetrahydrofuran-3yl), tetrahydrothiophenyl (eg tetrahydrothiophen-2-yl), thietanyl (eg thietan-3-yl), 1,3-dithiolanyl (eg 1,3-dithiothan-2-yl), 1,3-dioxolanyl (eg 1,3-dioxolan-2-yl) and pyrrolidinyl, and substituted derivatives thereof eg 2-methyltetrahydrofuran-2-yl, 3-methyl-tetrahydrofuran-2-yl and N-methyl-2-pyrrolidinyl. Other examples of substituted derivatives include 2-methyl-1-3,-dioxolan-2-yl, 2-methyl-1-3,-dithiolan-2-yl, 2-methyl-tetrahydrothiophen-2-yl, 3-methyl-tetrahydrofuran-3-yl. A further example of group R₂ is 2-ethyl-tetrahydrofuran-2-yl. Examples of 6 membered groups include 1,3-dithian-2-yl and tetrahydro-4H-pyran-4-yl.

We prefer that R₂ represents a 4-7 membered non-aromatic ring containing 1-3 heteroatoms selected from O, N and S optionally substituted with one or more methyl, ethyl or halogen groups. Generally we prefer that R₂ is not substituted or is substituted by 1 methyl group, and especially is not substituted. However one class of compounds of particular interest is that in which R₂ is substituted by methyl at the point of attachment to the carbonyl moiety (as exemplified, for example, by 2-methyl-1,3-dioxolan-2-yl, 2-methyl-tetrahydrofuran-2-yl, 2-methyl-tetrahydrothiophen-2-yl, 3-methyl-tetrahydrofuran-3-yl, 3-methyl-tetrahydrothiophen-3-yl). Preferably a heteroatom is present in the 2-position.

We prefer R₃ to represent methyl, especially methyl in the α configuration.

Compounds of formula (I) in which R₄ and R₅, which can be the same or different, each represents hydrogen, fluorine or chlorine, particularly hydrogen or fluorine, are preferred. Especially preferred are compounds in which both R₄ and R₅ are fluorine.

Preferably, -̅-̅-̅-̅-̅ represents a double bond.

It is to be understood that the present invention covers all combinations of particularly and preferred groups referred to hereinabove.

Preferred compounds of formula (I) include:
6α,9α-Difluoro-11β-hydroxy-16α-methyl-3-oxo-17α-(tetrahydrofuran-2S-ylcarbonyl)oxy-androsta-1,4-diene-17β-carbothioic acid S-fluoromethyl ester;
6α,9α-Difluoro-11β-hydroxy-16α-methyl-3-oxo-17α-(tetrahydrofuran-2R-ylcarbonyl)oxy-androsta-1,4-diene-17β-carbothioic acid S-fluoromethyl ester;
6α,9α-Difluoro-11β-hydroxy-16α-methyl-3-oxo-17α-(tetrahydrofuran-3-ylcarbonyl)oxy-androsta-1,4-diene-17β-carbothioic acid S-fluoromethyl ester;
6α,9α-Difluoro-17α-(1,3-dithiolan-2-ylcarbonyl)oxy-11β-hydroxy-16α-methyl-3-oxo-androsta-1,4-diene-17β-carbothioic acid S-fluoromethyl ester;
6α,9α-Difluoro-11β-hydroxy-16α-methyl-17α-(1-methyl-L-prolyl)oxy-3-oxo-androsta-1,4-diene-17β-carbothioic acid S-fluoromethyl ester;
6α,9α-Difluoro-11β-hydroxy-16α-methyl-3-oxo-17α-(thietan-3-ylcarbonyl)oxy-androsta-1,4-diene-17β-carbothioic acid S-fluoromethyl ester;
6α,9α-Difluoro-11β-hydroxy-16α-methyl-3-oxo-17α-(tetrahydrothiophen-2-ylcarbonyl)oxy-androsta-1,4-diene-17β-carbothioic acid S-fluoromethyl ester;
6α,9α-Difluoro-11β-hydroxy-16α-methyl-3-oxo-17α-(2-methyl-tetrahydrofuran-2R-ylcarbonyl)oxy-androsta-1,4-diene-17β-carbothioic acid S-fluoromethyl ester;
6α,9α-Difluoro-11β-hydroxy-16α-methyl-3-oxo-17α-(2-methyl-tetrahydrofuran-2S-ylcarbonyl)oxy-androsta-1,4-diene-17β-carbothioic acid S-fluoromethyl ester;
6α,9α-Difluoro-17α-(1,3-dioxolan-2-ylcarbonyl)oxy-11β-hydroxy-16α-methyl-3-oxo-androsta-1,4-diene-17β-carbothioic acid S-fluoromethyl ester;
6α,9α-Difluoro-11β-hydroxy-16α-methyl-3-oxo-17α-[(2R,3S)-3-methyltetrahydrofuran-2-ylcarbonyl]oxy-androsta-1,4-diene-17β-carbothioic acid S-fluoromethyl ester;
6α,9α-Difluoro-11β-hydroxy-16α-methyl-3-oxo-17α-[(2S,3R)-3-methyltetrahydrofuran-2-ylcarbonyl]oxy-androsta-1,4-diene-17β-carbothioic acid S-fluoromethyl ester;
6α,9α-Difluoro-11β-hydroxy-16α-methyl-17α-(2-methyl-1,3-dioxolan-2-ylcarbonyl)oxy-3-oxo-androsta-1,4-diene-17β-carbothioic acid S-fluoromethyl ester;
6α,9α-Difluoro-11β-hydroxy-16α-methyl-17α-(2-methyl-1,3-dithiolan-2-ylcarbonyl)oxy-3-oxo-androsta-1,4-diene-17β-carbothioic acid S-fluoromethyl ester;
6α,9α-Difluoro-11β-hydroxy-16α-methyl-3-oxo-17α-(2-methyl-tetrahydrothiophen-2R-ylcarbonyl)oxy-androsta-1,4-diene-17β-carbothioic acid S-fluoromethyl ester;
6α,9α-Difluoro-11β-hydroxy-16α-methyl-3-oxo-17α-(2-methyl-tetrahydrothiophen-2S-ylcarbonyl)oxy-androsta-1,4-diene-17β-carbothioic acid S-fluoromethyl ester;
6α,9α-Difluoro-11β-hydroxy-16α-methyl-3-oxo-17α-(3-methyltetrahydrofuran-3R-ylcarbonyl)oxy-androsta-1,4-diene-17β-carbothioic acid S-fluoromethyl ester;
6α,9α-Difluoro-11β-hydroxy-16α-methyl-3-oxo-17α-(3-methyltetrahydrofuran-3S-ylcarbonyl)oxy-androsta-1,4-diene-17β-carbothioic acid S-fluoromethyl ester;
6α,9α-Difluoro-11β-hydroxy-16α-methyl-3-oxo-17α-(2-ethyl-tetrahydrofuran-2R-ylcarbonyl)oxy-androsta-1,4-diene-17β-carbothioic acid S-fluoromethyl ester;
6α,9α-Difluoro-11β-hydroxy-16α-methyl-3-oxo-17α-(2-ethyl-tetrahydrofuran-2S-ylcarbonyl)oxy-androsta-1,4-diene-17β-carbothioic acid S-fluoromethyl ester;
6α,9α-Difluoro-17α-(1,3-dithian-2-ylcarbonyl)oxy-11β-hydroxy-16α-methyl-3-oxo-androsta-1,4-diene-17β-carbothioic acid S-fluoromethyl ester;
6α,9α-Difluoro-11β-hydroxy-16α-methyl-3-oxo-17α-(tetrahydro-4H-pyran-4-ylcarbonyl)oxy-androsta-1,4-diene-17β-carbothioic acid S-fluoromethyl ester;
and salts and solvated theref.

The compounds of formula (I) have potentially beneficial anti-inflammatory or anti-allergic effects, particularly upon topical administration, demonstrated by, for example, their ability to bind to the glucocorticoid receptor and to illicit a response via that receptor with long lasting effect. Hence, the compounds of formula (I) are useful in the treatment of inflammatory and/or allergic disorders, especially in once-per-day therapy.

Examples of disease states in which the compounds of the invention have utility include skin diseases such as eczema, psoriasis, allergic dermatitis neurodermatitis, pruritis and hypersensitivity reactions; inflammatory conditions of the nose, throat or lungs such as asthma (including allergen-induced asthmatic reactions), rhinitis (including hayfever), nasal polyps, chronic obstructive pulmonary disease, interstitial lung disease, and fibrosis; inflammatory bowel conditions such as ulcerative colitis and Crohn's disease; and auto-immune diseases such as rheumatoid arthritis.

Compounds of the invention may also have use in the treatment of conjunctiva and conjunctivitis.

It will be appreciated by those skilled in the art that reference herein to treatment extends to prophylaxis as well as the treatment of established conditions.

As mentioned above, compounds of formula (I) are useful in human or veterinary medicine, in particular as anti-inflammatory and anti-allergic agents, especially in once-per-day therapy.

There is thus provided as a further aspect of the invention a compound of formula (I) or a physiologically acceptable salt or solvate thereof for use in human or veterinary medicine, particularly in the treatment of patients with inflammatory and/or allergic conditions, especially for treatment once-per-day.

According to another aspect of the invention, there is provided the use of a compound of formula (I) or physiologically acceptable salt or solvate thereof for the manufacture of a medicament for the treatment of patients with inflammatory and/or allergic conditions, especially for treatment once-per-day.

The compounds according to the invention may be formulated for administration in any convenient way, and the invention therefore also includes within its scope pharmaceutical compositions comprising a compound of formula (I) or physiologically acceptable salt or solvate thereof together, if desirable, in admixture with one or more physiologically acceptable diluents or carriers. Pharmaceutical compositions for once-per-day administration are of particular interest.

Further, there is provided a process for the preparation of such pharmaceutical compositions which comprises mixing the ingredients.

The compounds according to the invention may, for example, be formulated for oral, buccal, sublingual, parenteral, local or rectal administration, especially local administration.

Local administration as used herein, includes administration by insufflation and inhalation. Examples of various types of preparation for local administration include ointments, lotions, creams, gels, foams, preparations for delivery by transdermal patches, powders, sprays, aerosols, capsules or cartridges for use in an inhaler or insufflator or drops (e.g. eye or nose drops), solutions/suspensions for nebulisation, suppositories, pessaries, retention enemas and chewable or suckable tablets or pellets (e.g. for the treatment of aphthous ulcers) or liposome or microencapsulation preparations.

Advantageously compositions for topical administration to the lung include dry powder compositions and spray compositions.

Dry powder compositions for topical delivery to the lung may, for example, be presented in capsules and cartridges for use in an inhaler or insufflator of, for example, gelatine. Formulations generally contain a powder mix for inhalation of the compound of the invention and a suitable powder base such as lactose or starch. Use of lactose is preferred. Each capsule or cartridge may generally contain between 20µg-10mg of the compound of formula (I) optionally in combination with another active ingredient. Alternatively, the compound of the invention may be presented without excipients. Packaging of the formulation may be suitable for unit dose or multi-dose delivery. In the case of multi-dose delivery, the formulation can be pre-metered (eg as in Diskus, see GB 2242134 or Diskhaler, see GB 2178965, 2129691 and 2169265) or metered in use (eg as in Turbuhaler, see EP 69715). An example of a unit-dose device is Rotahaler (see GB 2064336). The Diskus inhalation device comprises an elongate strip formed from a base sheet having a plurality of recesses spaced along its length and a lid sheet hermetically but peelably sealed thereto to define a plurality of containers, each container having therein an inhalable formulation containing a compound of formula (1) preferably combined with lactose. Preferably, the strip is sufficiently flexible to be wound into a roll. The lid sheet and base sheet will preferably have leading end portions which are not sealed to one another and at least one of the said leading end portions is constructed to be attached to a winding means. Also, preferably the hermetic seal between the base and lid sheets extends over their whole width. The lid sheet may preferably be peeled from the base sheet in a longitudinal direction from a first end of the said base sheet.

Pharmaceutical formulations which are non-pressurised and adapted to be administered as a dry powder topically to the lung via the buccal cavity (especially those which are free of excipient or are formulated with a diluent or carrier such as lactose or starch, most especially lactose) are of particular interest.

Spray compositions may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the compound of formula (I) and a suitable propellant such as a fluorocarbon or hydrogen-containing chlorofluorocarbon or mixtures thereof, particularly hydrofluoroalkanes, especially 1,1,1,2-tetrafluoroethane, 1,1,1,2,3,3,3-heptafluoro-n-propane or a mixture thereof. The aerosol composition may optionally contain additional formulation excipients well known in the art such as surfactants eg oleic acid or lecithin and cosolvents eg ethanol. One example formulation is excipient free and consists essentially of (eg consists of) compound of formula (I) (preferably in unsolvated form) (optionally in combination with another therapeutically active ingredient) and a propellant selected from 1,1,1,2-tetrafluoroethane, 1,1,1,2,3,3,3-heptafluoro-n-propane and mixture thereof. Another example formulation comprises particulate compound of formula (I), a propellant selected from 1,1,1,2-tetrafluoroethane, 1,1,1,2,3,3,3-heptafluoro-n-propane and mixture thereof and a suspending agent which is soluble in the propellant eg an oligolactic acid or derivative thereof as described in WO94/21229. The preferred propellant is 1,1,1,2-tetrafluoroethane. Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10µm, preferably 2-5µm. Particles having a size above 20µm are generally too large when inhaled to reach the small airways. To achieve these particle sizes the particles of compound of formula (I) as produced may be size reduced by conventional means eg by micronisation. The desired fraction may be separated out by air classification or sieving. Preferably, the particles will be crystalline, prepared for example by a process which comprises mixing in a continuous flow cell in the presence of ultrasonic radiation a flowing solution of compound of formula (I) as medicament in a liquid solvent with a flowing liquid antisolvent for said medicament (eg as described in International Patent Application PCT/GB99/04368) or else by a process which comprises admitting a stream of solution of the substance in a liquid solvent and a stream of liquid antisolvent for said substance tangentially into a cylindrical mixing chamber having an axial outlet port such that said streams are thereby intimately mixed through formation of a vortex and precipitation of crystalline particles of the substance is thereby caused (eg as described in International Patent Application PCT/GB00/04327). When an excipient such as lactose is employed, generally, the particle size of the excipient will be much greater than the inhaled medicament within the present invention. When the excipient is lactose it will typically be present as milled lactose, wherein not more than 85% of lactose particles will have a MMD of 60-90µm and not less than 15% will have a MMD of less than 15µm.

Formulations for administration topically to the nose (eg for the treatment of rhinitis) include pressurised aerosol formulations and aqueous formulations administered to the nose by pressurised pump. Formulations which are non-pressurised and adapted to be administered topically to the nasal cavity are of particular interest. The formulation preferably contains water as the diluent or carrier for this purpose. Aqueous formulations for administration to the lung or nose may be provided with conventional excipients such as buffering agents, tonicity modifying agents and the like. Aqueous formulations may also be administered to the nose by nebulisation.

Other possible presentations include the following:
Ointments, creams and gels, may, for example, be formulated with an aqueous or oily base with the addition of suitable thickening and/or gelling agent and/or solvents. Such bases may thus, for example, include water and/or an oil such as liquid paraffin or a vegetable oil such as arachis oil or castor oil, or a solvent such as polyethylene glycol. Thickening agents and gelling agents which may be used according to the nature of the base include soft paraffin, aluminium stearate, cetostearyl alcohol, polyethylene glycols, woolfat, beeswax, carboxypolymethylene and cellulose derivatives, and/or glyceryl monostearate and/or non-ionic emulsifying agents.

Lotions may be formulated with an aqueous or oily base and will in general also contain one or more emulsifying agents, stabilising agents, dispersing agents, suspending agents or thickening agents.

Powders for external application may be formed with the aid of any suitable powder base, for example, talc, lactose or starch. Drops may be formulated with an aqueous or non-aqueous base also comprising one or more dispersing agents, solubilising agents, suspending agents or preservatives.

Advantageously, the formulations of the invention may be buffered by the addition of suitable buffering agents.

The proportion of the active compound of formula (I) in the local compositions according to the invention depends on the precise type of formulation to be prepared but will generally be within the range of from 0.001 to 10% by weight. Generally, however for most types of preparations advantageously the proportion used will be within the range of from 0.005 to 1 % and preferably 0.01 to 0.5%. However, in powders for inhalation or insufflation the proportion used will usually be within the range of from 0.1 to 5%.

Aerosol formulations are preferably arranged so that each metered dose or "puff' of aerosol contains 20µg-2000µg, preferably about 20µg-500µg of a compound of formula (I) optionally in combination with another therapeutically active ingredient. Administration may be once daily or several times daily, for example 2, 3, 4 or 8 times, giving for example 1, 2 or 3 doses each time. The overall daily dose with an aerosol will be within the range 100µg-10mg preferably, 200µg-2000µg. The overall daily dose and the metered dose delivered by capsules and cartridges in an inhaler or insufflator will generally be double those with aerosol formulations.

Since the compounds of formula (I) are long-acting, preferably the compound will be delivered once-per-day and the dose will be selected so that the compound has a therapeutic effect in the treatment of respiratory disorders (eg asthma or COPD, particularly asthma) over 24 hours or more.

Topical preparations may be administered by one or more applications per day to the affected area; over skin areas occlusive dressings may advantageously be used. Continuous or prolonged delivery may be achieved by an adhesive reservoir system.

For internal administration the compounds according to the invention may, for example, be formulated in conventional manner for oral, parenteral or rectal administration. Formulations for oral administration include syrups, elixirs, powders, granules, tablets and capsules which typically contain conventional excipients such as binding agents, fillers, lubricants, disintegrants, wetting agents, suspending agents, emulsifying agents, preservatives, buffer salts, flavouring, colouring and/or sweetening agents as appropriate. Dosage unit forms are, however, preferred as described below.

Preferred forms of preparation for internal administration are dosage unit forms i.e. tablets and capsules. Such dosage unit forms contain from 0.1mg to 20mg preferably from 2.5 to 10mg of the compounds of the invention.

The compounds according to the invention may in general may be given by internal administration in cases where systemic adreno-cortical therapy is indicated.

In general terms preparations, for internal administration may contain from 0.05 to 10% of the active ingredient dependent upon the type of preparation involved. The daily dose may vary from 0.1 mg to 60mg, e.g. 5-30mg, dependent on the condition being treated, and the duration of treatment desired.

Slow release or enteric coated formulations may be advantageous, particularly for the treatment of inflammatory bowel disorders.

The pharmaceutical compositions according to the invention may also be used in combination with another therapeutically active agent, for example, a β₂ adrenoreceptor agonist, an anti-histamine or an anti-allergic. The invention thus provides, in a further aspect, a combination comprising the compound of formula (I) or a physiologically acceptable salt or solvate thereof together with another therapeutically active agent, for example, a β₂-adrenoreceptor agonist, an anti-histamine or an anti-allergic.

Examples of β₂-adrenoreceptor agonists include salmeterol (eg as racemate or single enantiomer such as the s-enantiomer), salbutamol, formoterol, fenoterol or terbutaline and salts thereof, for example the xinafoate salt of salmeterol, the sulphate salt or free base of salbutamol or the fumarate salt of formoterol. Long-acting β₂-adrenoreceptor agonists are preferred, especially those having a therapeutic effect over a 24 hour period.

Especially preferred long-acting β₂-adrenoreceptor agonists are compounds of formula (X) 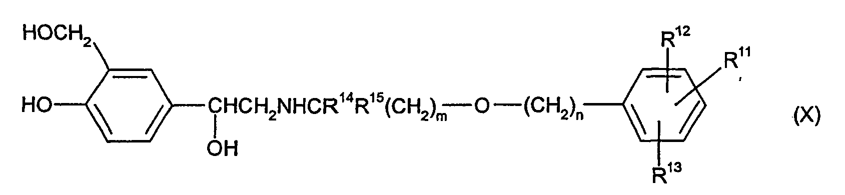 or a salt or solvate thereof, wherein:
m is an integer of from 2 to 8;
n is an integer of from 3 to 11, preferably from 3 to 7;
with the proviso that m + n is 5 to 19, preferably 5 to 12;
R¹¹ is -XSO₂NR¹⁶R¹⁷
wherein X is -(CH₂)ₚ- or C₂₋₆ alkenylene;
R¹⁶ and R¹⁷ are independently selected from hydrogen, C₁₋₆alkyl, C₃₋₇cycloalkyl, C(O)NR¹⁸R¹⁹, phenyl, and phenyl (C₁₋₄alkyl)-,
or R¹⁶ and R¹⁷, together with the nitrogen to which they are bonded, form a 5-, 6-, or 7- membered nitrogen containing ring, and R¹⁶ and R¹⁷ are each optionally substituted by one or two groups selected from halo, C₁₋₆alkyl, C₁₋₆haloalkyl, C₁₋₆alkoxy, hydroxy-substituted C₁₋₆alkoxy, -CO₂R¹⁸, -SO₂NR¹⁸R¹⁹, -CONR¹⁸R¹⁹,-NR¹⁸C(O)R¹⁹, or a 5-, 6- or 7-membered heterocylic ring;
R¹⁸ and R¹⁹ are independently selected from hydrogen, C₁₋₆alkyl, C₃₋₆cycloalkyl, phenyl, and phenyl (C₁₋₄alkyl)-; and
p is an integer of from 0 to 6, preferably from 0 to 4;
R¹² and R¹³ are independently selected from hydrogen, C₁₋₆alkyl, C₁₋₆alkoxy, halo, phenyl, and C₁₋₆haloalkyl; and
R¹⁴ and R¹⁵ are independently selected from hydrogen and C₁₋₄alkyl with the proviso that the total number of carbon atoms in R¹⁴ and R¹⁵ is not more than 4.
In the compounds of formula (I) the group R¹¹ is preferably attached to the meta-position relative to the -O-(CH₂)ₙ- link.
R¹¹ preferably represents -SO₂NR¹⁶R¹⁷ wherein R¹⁶ and R¹⁷ are independently selected from hydrogen and C₁₋₆alkyl, more preferably R¹¹ is -SO₂NH₂.
R¹⁴ and R¹⁵ are preferably independently selected from hydrogen and methyl, more preferably R¹⁴ and R¹⁵ are both hydrogen.
m is suitably 4, 5, or 6, and n is suitably 3, 4, 5 or 6. Preferably m is 5 or 6 and n is 3 or 4, such that m + n is 8, 9 or 10, preferably 9.

More especially preferred compounds of formula (X) are compounds of formula (Xa) 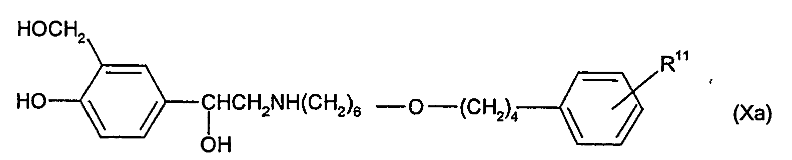 or a salt or solvate thereof, wherein
R¹¹ is as defined above for formula (X).

Further more especially preferred compounds of formula (X) are compounds of formula (Xb): 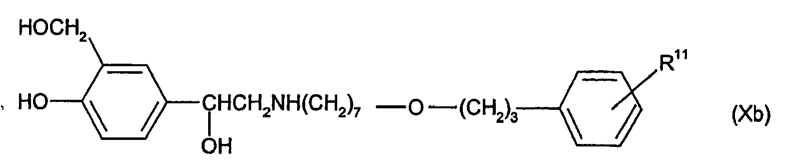 or a salt or solvate thereof, wherein
R¹¹ is as defined above for formula (X).

In the compounds of formulae (Xa) and (Xb), the group R¹¹ is preferably attached to the meta-position relative to the -O-(CH₂)₄- or -O-(CH₂)₃- link respectively.

In the compounds of formulae (Xa) and (Xb), R¹¹ is preferably -SO₂NR¹⁶R¹⁷ wherein R¹⁶ and R¹⁷ are independently selected from hydrogen and C₁₋₆alkyl, more preferably R¹¹ is -SO₂NH₂.

In the definition of R¹¹ where 'R¹⁶ and R¹⁷ together with the nitrogen atom to which they are bonded, form a 5-, 6-, or 7- membered nitrogen containing ring', the term "5-, 6-, or 7- membered nitrogen containing ring" means a 5-, 6-, or 7-membered saturated or unsaturated ring which includes the sulfonamide nitrogen atom and optionally 1 or 2 other heteroatoms independently selected from nitrogen, sulphur, and oxygen. Suitable examples of such a ring include piperidinyl, morpholinyl, and piperazinyl.

In the definition of R¹¹, specifically the optional substituents on R¹⁶ and R¹⁷, the term "5-, 6-, or 7- membered heterocyclic ring" means a 5-, 6-, or 7- membered fully or partially saturated or unsaturated ring which includes 1, 2, 3 or 4 heteroatoms independently selected from nitrogen, sulphur, and oxygen. Suitable examples of such a ring include pyrrolyl, furyl, thienyl, pyridinyl, pyrazinyl, pyridazinyl, imidazolyl, tetrazolyl, tetrahydrofuranyl, oxazolyl, thiazolyl, thiadiazolyl, piperidinyl, morpholinyl, and piperazinyl.

In the definition of X, the term "alkenylene" includes both *cis* and *trans* structures. Suitable examples of alkenylene groups include -CH=CH-.

The compounds of formulae (X), (Xa) and (Xb) include an asymmetric centre, namely the carbon atom of the 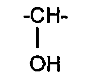 group. The present invention includes both (S) and (R) enantiomers either in substantially pure form or admixed in any proportions.

Similarly, where R¹⁴ and R¹⁵ are different groups, the carbon atom to which they are attached is an asymmetric centre and the present invention includes both (S) and (R) enantiomers at this centre either in substantially pure form or admixed in any proportions.

Thus the compounds of formulae (X), (Xa) and (Xb) include all enantiomers and diastereoisomers as well as mixtures thereof in any proportions.

The most preferred compound of formula (X) is 3-(4-{[6-({(2R)-2-Hydroxy-2-[4-hydroxy-3-(hydroxymethyl)phenyl]ethyl}amino)-hexyl]oxy}butyl)benzenesulfonamide or a salt or solvate thereof.

Salts and solvates of compounds of formulae (X), (Xa) and (Xb) which are suitable for use in medicine are those wherein the counterion or associated solvent is pharmaceutically acceptable. However, salts and solvates having non-pharmaceutically acceptable counterions or associated solvents are within the scope of the present invention, for example, for use as intermediates in the preparation of other compounds of formulae (X), (Xa) and (Xb) and their pharmaceutically acceptable salts and solvates.

Suitable salts according to the invention include those formed with both organic and inorganic acids or bases. Pharmaceutically acceptable acid addition salts include those formed from hydrochloric, hydrobromic, sulphuric, citric, tartaric, phosphoric, lactic, pyruvic, acetic, trifluoroacetic, triphenylacetic, sulphamic, sulphanilic, succinic, oxalic, fumaric, maleic, malic, glutamic, aspartic, oxaloacetic, methanesulphonic, ethanesulphonic, arylsulphonic (for example p-toluenesulphonic, benzenesulphonic, naphthalenesulphonic or naphthalenedisulphonic), salicylic, glutaric, gluconic, tricarballylic, cinnamic, substituted cinnamic (for example, phenyl, methyl , methoxy or halo substituted cinnamic, including 4-methyl and 4-methoxycinnamic acid), ascorbic, oleic, naphthoic, hydroxynaphthoic (for example 1- or 3-hydroxy-2-naphthoic), naphthaleneacrylic (for example naphthalene-2-acrylic), benzoic, 4-methoxybenzoic, 2- or 4-hydroxybenzoic, 4-chlorobenzoic, 4-phenylbenzoic, benzeneacrylic (for example 1,4-benzenediacrylic) and isethionic acids. Pharmaceutically acceptable base salts include ammonium salts, alkali metal salts such as those of sodium and potassium, alkaline earth metal salts such as those of calcium and magnesium and salts with organic bases such as dicyclohexyl amine and N-methyl-D-glucamine.

Compounds of formula (X), (Xa) and (Xb) may be prepared by reference to Example X recited below, by analogous processes, or by other conventional processes known *per se*.

Since the compounds of formula (I) are long-acting, preferably the composition comprising the compound of formula (I) and the long-acting β₂-adrenoreceptor agonists will be delivered once-per-day and the dose of each will be selected so that the composition has a therapeutic effect in the treatment of respiratory disorders effect (eg in the treatment of asthma or COPD, particularly asthma) over 24 hours or more.

Examples of anti-histamines include methapyrilene or loratadine. Examples of anti-allergics include cromoglycate (eg as sodium), ketotifen and nedocromil (as as sodium). Examples of anti-cholinergics include ipratropium (eg as bromide), tiotropium, atropine or oxitropium. Any of the aforementioned substances may be employed in the form of alternative salts or solvates thereof.

Other suitable combinations include, for example, other anti-inflammatory agents eg. NSAIDs (eg. sodium cromoglycate, nedocromil sodium, PDE4 inhibitors, leukotriene antagonists, iNOS inhibitors, tryptase and elastase inhibitors, beta-2 integrin antagonists and adenosine 2a agonists)) or antiinfective agents (eg. antibiotics, antivirals).

Of particular interest is use of the compound of formula (I) in combination with a phosphodiesterase 4 (PDE4) inhibitor. The PDE4-specific inhibitor useful in this aspect of the invention may be any compound that is known to inhibit the PDE4 enzyme or which is discovered to act as a PDE4 inhibitor, and which are only PDE4 inhibitors, not compounds which inhibit other members of the PDE family as well as PDE4. Generally it is preferred to use a PDE4 inhibitor which has an IC₅₀ ratio of about 0.1 or greater as regards the IC₅₀ for the PDE4 catalytic form which binds rolipram with a high affinity divided by the IC₅₀ for the form which binds rolipram with a low affinity. For the purposes of this disclosure, the cAMP catalytic site which binds R and S rolipram with a low affinity is denominated the "low affinity" binding site (LPDE 4) and the other form of this catalytic site which binds rolipram with a high affinity is denominated the "high affinity" binding site (HPDE 4). This term "HPDE4" should not be confused with the term "hPDE4" which is used to denote human PDE4.
Initial experiments were conducted to establish and validate a [³H]-rolipram binding assay. Details of this work are given in the Binding Assays described in detail below.

The preferred PDE4 inhibitors of use in this invention will be those compounds which have a salutary therapeutic ratio, i.e., compounds which preferentially inhibit cAMP catalytic activity where the enzyme is in the form that binds rolipram with a low affinity, thereby reducing the side effects which apparently are linked to inhibiting the form which binds rolipram with a high affinity. Another way to state this is that the preferred compounds will have an IC₅₀ ratio of about 0.1 or greater as regards the IC₅₀ for the PDE4 catalytic form which binds rolipram with a high affinity divided by the IC₅₀ for the form which binds rolipram with a low affinity.
A further refinement of this standard is that of one wherein the PDE4 inhibitor has an IC₅₀ ratio of about 0.1 or greater; said ratio is the ratio of the IC₅₀ value for competing with the binding of 1nM of [³H]R-rolipram to a form of PDE4 which binds rolipram with a high affinity over the IC₅₀ value for inhibiting the PDE4 catalytic activity of a form which binds rolipram with a low affinity using 1 µM[³H]-cAMP as the substrate.

Examples of useful PDE4 inhibitors are:
(R)-(+)-1-(4-bromobenzyl)-4-[(3-cyclopentyloxy)-4-methoxyphenyl]-2-pyrrolidone;
(R)-(+)-1-(4-bromobenzyl)-4-[(3-cyclopentyloxy)-4-methoxyphenyl]-2-pyrrolidone;
3-(cyclopentyloxy-4-methoxyphenyl)-1-(4-N'-[N2-cyano-S-methyl-isothioureido]benzyl)-2-pyrrolidone;
cis 4-cyano-4-(3-cyclopentyloxy-4-methoxyphenyl)cyclohexan-1-carboxylic acid];
cis-[4-cyano-4-(3-cyclopropylmethoxy-4-difluoromethoxyphenyl)cyclohexan-1-ol];
(R)-(+)-ethyl [4-(3-cyclopentyloxy-4-methoxyphenyl)pyrrolidine-2-ylidene]acetate; and
(S)-(-)-ethyl [4-(3-cyclopentyloxy-4-methoxyphenyl)pyrrolidine-2-ylidene]acetate.

Most preferred are those PDE4 inhibitors which have an IC₅₀ ratio of greater than 0.5, and particularly those compounds having a ratio of greater than 1.0. Preferred compounds are *cis* 4-cyano-4-(3-cyclopentyloxy-4-methoxyphenyl)cyclohexan-1-carboxylic acid, 2-carbomethoxy-4-cyano-4-(3-cyclopropylmethoxy-4-difluoromethoxyphenyl)cyclohexan-1-one and cis-[4-cyano-4-(3-cyclopropylmethoxy-4-difluoromethoxyphenyl)cyclohexan-1-ol]; these are examples of compounds which bind preferentially to the low affinity binding site and which have an IC₅₀ ratio of 0.1 or greater.
Other compounds of interest include:
Compounds set out in U.S. patent 5,552,438 issued 03 September, 1996; this patent and the compounds it discloses are incorporated herein in full by reference. The compound of particular interest, which is disclosed in U.S. patent 5,552,438, is *cis*-4-cyano-4-[3- (cyclopentyloxy)-4-methoxyphenyl]cyclohexane-1-carboxylic acid (also known as cilomalast) and its salts, esters, pro-drugs or physical forms;
AWD-12-281 from Astra (Hofgen, N. et al. 15th EFMC Int Symp Med Chem (Sept 6-10, Edinburgh) 1998, Abst P.98); a 9-benzyladenine derivative nominated NCS-613 (INSERM); D-4418 from Chiroscience and Schering-Plough; a benzodiazepine PDE4 inhibitor identified as CI-1018 (PD-168787; Parke-Davis/Warner-Lambert); a benzodioxole derivative Kyowa Hakko disclosed in WO 9916766; V-11294A from Napp (Landells, L.J. et al. Eur Resp J [Annu Cong Eur Resp Soc (Sept 19-23, Geneva) 1998] 1998, 12(Suppl. 28): Abst P2393); roflumilast (CAS reference No 162401-32-3) and a pthalazinone (WO 9947505) from Byk-Gulden; or a compound identified as T-440 (Tanabe Seiyaku; Fuji, K. et al. J Pharmacol Exp Ther,1998, 284(1): 162).

### Phosphodiesterase and Rolipram Binding Assays

### Assay method 1A

Isolated human monocyte PDE4 and hrPDE (human recombinant PDE4) was determined to exist primarily in the low affinity form. Hence, the activity of test compounds against the low affinity form of PDE4 can be assessed using standard assays for PDE4 catalytic activity employing 1 µM [³H]cAMP as a substrate (Torphy et al., J. of Biol. Chem., Vol. 267, No. 3 pp1798-1804, 1992).
Rat brain high speed supernatants were used as a source of protein and both enantiomers of [³H]-rolipram were prepared to a specific activity of 25.6 Ci/mmol. Standard assay conditions were modified from the published procedure to be identical to the PDE assay conditions, except for the last of the cAMP: 50mM Tris HCI (pH 7.5), 5 mM MgCl₂, 50 µM 5'-AMP and 1 nM of [³H]-rolipram (Torphy et al., J. of Biol. Chem., Vol. 267, No. 3 pp1798-1804, 1992). The assay was run for 1 hour at 30° C. The reaction was terminated and bound ligand was separated from free ligand using a Brandel cell harvester. Competition for the high affinity binding site was assessed under conditions that were identical to those used for measuring low affinity PDE activity, expect that [³H]-cAMP was not present.

### Assay method 1B

### Measurement of Phosphodiesterase Activity

PDE activity was assayed using a [³H]cAMP SPA or [³H]cGMP SPA enzyme assay as described by the supplier (Amersham Life Sciences). The reactions were conducted in 96-well plates at room temperature, in 0.1 ml of reaction buffer containing (final concentrations): 50 mM Tris-HCl, pH 7.5, 8.3 mM MgCl₂, 1.7 mM EGTA, [³H]cAMP or [³H] cGMP (approximately 2000 dpm/pmol), enzyme and various concentrations of the inhibitors. The assay was allowed to proceed for 1 hr and was terminated by adding 50 µl of SPA yttrium silicate beads in the presence of zinc sulfate. The plates were shaken and allowed to stand at room temperature for 20 min. Radiolabeled product formation was assessed by scintillation spectrometry.

### [³H]R-rolipram binding assay

The [³H]R-rolipram binding assay was performed by modification of the method of Schneider and co-workers, see Nicholson, et al., Trends Pharmacol. Sci., Vol. 12, pp.19-27 (1991) and McHale et al., Mol. Pharmacol., Vol. 39, 109-113 (1991). R-Rolipram binds to the catalytic site of PDE4 see Torphy et al., Mol. Pharmacol., Vol. 39, pp. 376-384 (1991). Consequently, competition for [³H]R-rolipram binding provides an independent confirmation of the PDE4 inhibitor potencies of unlabeled competitors. The assay was performed at 30°C for 1 hr in 0.5 µl buffer containing (final concentrations): 50 mM Tris-HCI, pH 7.5, 5 mM MgCl₂, 0.05% bovine serum albumin, 2 nM [³H]R-rolipram (5.7 x 104 dpm/pmol) and various concentrations of non-radiolabeled inhibitors. The reaction was stopped by the addition of 2.5 ml of ice-cold reaction buffer (without [³H]-R-rolipram) and rapid vacuum filtration (Brandel Cell Harvester) through Whatman GF/B filters that had been soaked in 0.3% polyethylenimine. The filters were washed with an additional 7.5 ml of cold buffer, dried, and counted via liquid scintillation spectrometry.

The invention thus provides, in a further aspect, a combination comprising the compound of formula (I) or a physiologically acceptable salt or solvate thereof together with a PDE4 inhibitor.

The combination referred to above may conveniently be presented for use in the form of a pharmaceutical formulation and thus pharmaceutical formulations comprising a combination as defined above together with a pharmaceutically acceptable diluent or carrier represent a further aspect of the invention.

The individual compounds of such combinations may be administered either sequentially or simultaneously in separate or combined pharmaceutical formulations. Appropriate doses of known therapeutic agents will be readily appreciated by those skilled in the art.

The compounds of formula (I) and salts and solvates thereof may be prepared by the methodology described hereinafter, constituting a further aspect of this invention.

A process according to the invention for preparing a compound of formula (I) comprises alkylation of a thioacid of formula (II) 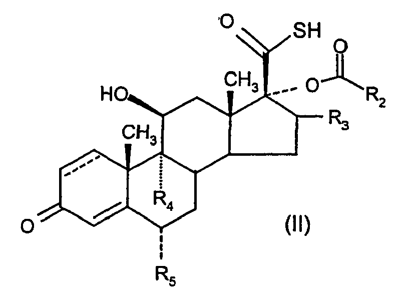 wherein R₂, R₃, R₄, R₅ and -̅-̅-̅-̅-̅ are as defined above.

In this process the compound of formula (II) may be reacted with, for example, an appropriate alkyl or haloalkyl halide under standard conditions.

When R₁ represents fluoromethyl, the preferred haloalkyl halide reagent is bromofluoromethane.

Compounds of formula (II) may be prepared from the corresponding 17α-hydroxyl derivative of formula (III): 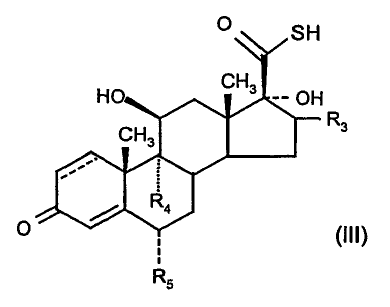 wherein R₂, R₃, R₄, R₅ and -̅-̅-̅-̅-̅ are as defined above, using for example, the methodology described by G. H. Phillipps et al., (1994) Journal of Medicinal Chemistry, 37, 3717-3729. For example the compound of formula (III) may be reacted with a compound of formula R₂COOH or an activated derivative thereof eg an activated ester, anhydride or halide (eg the acid chloride). The reaction may be performed in the presence of an organic solvent eg triethylamine, usually together with dimethylaminopyridine (DMAP).

Compounds of formula (III) may be prepared in accordance with procedures described in GB 2088877B. Compounds of formula (III) may also be prepared by a process comprising the following steps:

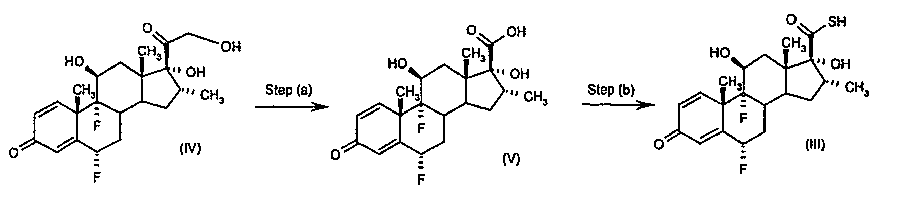

Step (a) comprises oxidation of a solution containing the compound of formula (IV). Preferably, step (a) will be performed in the presence of a solvent comprising methanol, water, tetrahydrofuran, dioxan or diethylene glygol dimethylether. For example, so as to enhance yield and throughput, preferred solvents are methanol, water or tetrahydrofuran, and more preferably are water or tetrahydrofuran, especially water and tetrahydrofuran as solvent. Dioxan and diethylene glygol dimethylether are also preferred solvents which may optionally (and preferably) be employed together with water. Preferably, the solvent will be present in an amount of between 3 and 10vol relative to the amount of the starting material (1wt.), more preferably between 4 and 6 vol., especially 5 vol. Preferably the oxidising agent is present in an amount of 1-9 molar equivalents relative to the amount of the starting material. For example, when a 50% w/w aqueous solution of periodic acid is employed, the oxidising agent may be present in an amount of between 1.1 and 10wt. relative to the amount of the starting material (1wt.), more preferably between 1.1 and 3wt., especially 1.3wt. Preferably, the oxidation step will comprise the use of a chemical oxidising agent. More preferably, the oxidising agent will be periodic acid or iodic acid or a salt thereof. Most preferably, the oxidising agent will be periodic acid or sodium periodate, especially periodic acid. Alternatively (or in addition), it will also be appreciated that the oxidation step may comprise any suitable oxidation reaction, eg. one which utilises air and/or oxygen. When the oxidation reaction utilises air and/or oxygen, the solvent used in said reaction will preferably be methanol. Preferably, step (a) will involve incubating the reagents at room temperature or a little warmer, say around 25 °C eg for 2 hours. The compound of formula (V) may be isolated by recrystallisation from the reaction mixture by addition of an anti-solvent. A suitable anti-solvent for compound of formula (V) is water. Surprisingly we have discovered that it is highly desirable to control the conditions under which the compound of formula (V) is precipitated by addition of anti-solvent eg water. When the recrystallisation is performed using chilled water (eg water/ice mixture at a temperature of 0-5 °C) although better anti-solvent properties may be expected we have found that the crystalline product produced is very voluminous, resembles a soft gel and is very difficult to filter. Without being limited by theory we believe that this low density product contains a large amount of solvated solvent within the crystal lattice By contrast when conditions of around 10 °C or higher are used (eg around ambient temperature) a granular product of a sand like consistency which is very easily filtered is produced. Under these conditions, crystallisation typically commences after around 1 hour and is typically completed within a few hours (eg 2 hours). Without being limited by theory we believe that this granular product contains little or no of solvated solvent within the crystal lattice.

Step (b) will typically comprise the addition of a reagent suitable for converting a carboxylic acid to a carbothioic acid eg. using hydrogen sulphide gas together with a suitable coupling agent eg. carbonyldiimidazole (CDI) in the presence of a suitable solvent eg. dimethylformamide.

Solvates of compounds of formula (I) which are not physiologically acceptable may be useful as intermediates in the preparation of compounds of formula (I) or physiologically acceptable solvates thereof.

The advantages of compounds "of formula (I) and/or salts and solvates thereof may include the fact that the substances appear to demonstrate excellent anti-inflammatory properties, with predictable pharmacokinetic and pharmacodynamic behaviour, with an attractive side-effect profile (demonstrated, for example, by good selectivity for the glucocorticoid receptor over the progesterone receptor and/or increased selectivity for glucocorticoid receptor mediated transrepression over transactivation) and are compatible with a convenient regime of treatment in human patients, especially for once-per-day administration. Further advantages may include the fact that the substances have desirable physical and chemical properties which allow for ready manufacture and storage.

The following non-limiting Examples illustrate the invention:

### EXAMPLES

### General

LCMS was conducted on a Supelcosil LCABZ+PLUS column (3.3 cm x 4.6 mm ID) eluting with 0.1% HCO₂H and 0.01 M ammonium acetate in water (solvent A), and 0.05% HCO₂H 5% water in acetonitrile (solvent B), using the following elution gradient 0-0.7 min 0%B, 0.7-4.2 min 100%B, 4.2-5.3 min 0%B, 5.3-5.5 min 0%B at a flow rate of 3 ml/min. The mass spectra were recorded on a Fisons VG Platform spectrometer using electrospray positive and negative mode (ES+ve and ES-ve).

### Intermediates

### Intermediate 1: 6α,9α-Difluoro-11β-hydroxy-16α-methyl-3-oxo-17α-(tetrahydrofuran-2S-ylcarbonyl)oxy-androsta-1,4-diene-17β-carbothioic acid

A solution of 6α,9α-difluoro-11β,17α-dihydroxy-16α-methyl-3-oxo-androsta-1,4-diene-17β-carbothioic acid (prepared in accordance with the procedure described in GB 2088877B) (1g, 2.42mmol) in anhydrous dichloromethane (20ml) and triethylamine (0.88ml, 6.32mmol) was treated at <5°C under nitrogen with a solution of S-tetrahydro-2-furoyl chloride (6.32mmol) in anhydrous dichloromethane (5ml) over approximately 2min. The solution was stirred at <5°C for 1h and then diluted with dichloromethane (20ml) and washed successively with 5% sodium hydrogen carbonate solution (20ml), 1 M hydrochloric acid (20ml) and water (20ml). The organic solution was dried (Na₂SO₄) and evaporated to give an cream foam (1.58g) which was dissolved in acetone (30ml) and treated with 1-methylpiperazine (1ml, 9mmol). After 2.5h the solution was slowly added to a stirred mixture of 2M hydrochloric acid (55ml) and ice (55ml) and the precipitate was collected and dried in vacuo to give the title compound as a white solid (1.08g, 87.4%): LCMS retention time 3.46min, *m*/*z* 511 MH⁺

### Intermediate 2: 6α,9α-Difluoro-11β-hydroxy-16α-methyl-3-oxo-17α-(tetrahydrofuran-2R-ylcarbonyl)oxy-androsta-1,4-diene-17β-carbothioic acid

Prepared using methods similar to that described for Intermediate 1.
LCMS retention time 3.45min, *m*/*z* 511 MH⁺

### Intermediate 3: 6α,9α-Difluoro-11β-hydroxy-16α-methyl-3-oxo-17α-(tetrahydrofuran-3-ylcarbonyl)oxy-androsta-1,4-diene-17β-carbothioic acid

Prepared using methods similar to that described for Intermediate 1.
LCMS retention time 3.39min, *m*/*z* 511 MH⁺

### Intermediate 4: 6α,9α-Difluoro-17α-(1,3-dithiolan-2-ylcarbonyl)oxy-11β-hydroxy-16α-methyl-3-oxo-androsta-1,4-diene-17β-carbothioic acid

Prepared using methods similar to that described for Intermediate 1.
LCMS retention time 3.75min, *m*/*z* 545 MH⁺

### Intermediate 5: 6α,9α-Difluoro-11β-hydroxy-16α-methyl-17α-(1-methyl-L-prolyl)oxy-3-oxo-androsta-1,4-diene-17β-carbothioic acid

Prepared using methods similar to that described for Intermediate 1.
LCMS retention time 2.56min, m/z 524 MH⁺

### Intermediate 6: 6α,9α-Difluoro-11β-hydroxy-16α-methyl-3-oxo-17α-(thietan-3-ylcarbonyl)oxy-androsta-1,4-diene-17β-carbothioic acid

Prepared using methods similar to that described for Intermediate 1.
LCMS retention time 3.73min, *m*/*z* 513 MH⁺

### Intermediate 7: 6α,9α-Difluoro-11β-hydroxy-16α-methyl-3-oxo-17α-(tetrahydrothiophen-2-ylcarbonyl)oxy-androsta-1,4-diene-17β-carbothioic acid

Prepared using methods similar to that described for Intermediate 1.
LCMS retention time 3.93min, *m*/*z* 527 MH⁺

### Intermediate 8: 6α,9α-Difluoro-11β-hydroxy-16α-methyl-3-oxo-17α-(2-methyltetrahydrofuran-2-ylcarbonyl)oxy-androsta-1,4-diene-17β-carbothioic acid

Prepared using methods similar to that described for Intermediate 1.
LCMS retention time 3.97min, *m*/*z* 525 MH⁺

### Intermediate 9:6α,9α=Difluoro-17α-(1,3-dioxolan-2-ylcarbonyl)oxy-11β-hydroxy-16α-methyl-3-oxo-androsta-1,4-diene-17β-carbothioic acid

Prepared using methods similar to that described for Intermediate 1.
LCMS retention time 3.27min, *m*/*z* 513 MH⁺

### Intermediate 10: 6α,9α-Difluoro-11β-hydroxy-16α-methyl-3-oxo-17α-[(2RS,3SR)-3-methyl-tetrahydrofuran-2-ylcarbonyl]oxy-androsta-1,4-diene-17β-carbothioic acid

Prepared using (2RS,3SR)-3-methyl-2-tetrahydrofuroic acid (prepared according to the method of WO 92/01696) and using a method similar to that described for Intermediate 1 LCMS retention time 3.77, 3.87min, *m*/*z* 525 MH⁺

### Intermediate 11: 6α,9α-Difluoro-11β-hydroxy-16α-methyl-17α-(2-methyl-1,3-dioxolan-2-ylcarbonyl)oxy-3-oxo-androsta-1,4-diene-17β-carbothioic acid

Prepared using methods similar to that described for Intermediate 1.
LCMS retention time 3.43min, *m*/*z* 527 MH⁺

### Intermediate 12: 6α,9α-Difluoro-11β-hydroxy-16α-methyl-17α-(2-methyl-1,3-dithiolan-2-ylcarbonyl)oxy-3-oxo-androsta-1,4-diene-17β-carbothioic acid

Prepared using methods similar to that described for Intermediate 1.
LCMS retention time 3.81 min, *m*/*z* 559 MH⁺

### Intermediate 13: 6α,9α-Difluoro-11β-hydroxy-16α-methyl-3-oxo-17α-(2-methyl-tetrahydrothiophen-2-ylcarbonyl)oxy-androsta-1,4-diene-17β-carbothioic acid

Prepared using methods similar to that described for Intermediate 1.
LCMS retention time 3.79min, *m*/*z* 541 MH⁺

### Intermediate 14: 6α,9α-Difluoro-11β-hydroxy-16α-methyl-3-oxo-17α-(3-methyltetrahydrofuran-3-ylcarbonyl)oxy-androsta-1,4-diene-17β-carbothioic acid

Prepared using methods similar to that described for Intermediate 1.
LCMS retention time 3.59min, *m*/*z* 525 MH⁺

### Intermediate 15: 6α,9α-Difluoro-11β-hydroxy-16α-methyl-3-oxo-17α-(2-ethyl-tetrahydrofuran-2-ylcarbonyl)oxy-androsta-1,4-diene-17β-carbothioic acid

Prepared using methods similar to that described for Intermediate 1.
LCMS retention time 3.92min, *m*/*z* 539 MH⁺

### Intermediate 16: 6α,9α-Difluoro-17α-(1,3-dithian-2-ylcarbonyl)oxy-11β-hydroxy-16α-methyl-3-oxo-androsta-1,4-diene-17β-carbothioic acid

Prepared using methods similar to that described for Intermediate 1.
LCMS retention time 4.29min, *m*/*z* 559 MH⁺

### Intermediate 17: 6α,9α-Difluoro-11β-hydroxy-16α-methyl-3-oxo-17α-(tetrahydro-4H-pyran-4-ylcarbonyl)oxy-androsta-1,4-diene-17β-carbothioic acid

Prepared using methods similar to that described for Intermediate 1.
LCMS retention time 3.53min, *m*/*z* 525 MH⁺

### Examples

### Example 1: 6α,9α-Difluoro-11β-hydroxy-16α-methyl-3-oxo-17α-(tetrahydrofuran-2S-ylcarbonyl)oxy-androsta-1,4-diene-17β-carbothioic acid S-fluoromethyl ester

Sodium hydrogen carbonate (109mg, 1.3mmol) was added to a solution of Intermediate 1 (600mg, 1.18mmol) in anhydrous N,N-dimethylformamide (6ml) and the mixture cooled to -20 °C under nitrogen. Bromofluoromethane (0.15ml, 2.7mmol) was added and the mixture was stirred at -20 °C for 2h. Diethylamine (0.6ml, 5.8mmol) was added and the mixture stirred at -20 °C for 15min and then added to vigorously stirred 2M hydrochloric acid (25ml). Water (75ml) was added and after stirring for a further 30min the white precipitate was collected and dried in vacuo (591 mg). This material was purified was column chromatography on silica gel to give the title compound as a white solid (307mg, 48%): LCMS retention time 3.41 min, *m*/*z* 543 MH⁺.

### Example 2: 6α,9α-Difluoro-11β-hydroxy-16α-methyl-3-oxo-17α-(tetrahydrofuran-2R-ylcarbonyl)oxy-androsta-1,4-diene-17β-carbothioic acid S-fluoromethyl ester

Prepared from Intermediate 2 using methods similar to that described for Example 1. LCMS retention time 3.38min, *m*/*z* 543 MH⁺

### Example 3: 6α,9α-Difluoro-11β-hydroxy-16α-methyl-3-oxo-17α-(tetrahydrofuran-3-ylcarbonyl)oxy-androsta-1,4-diene-17β-carbothioic acid S-fluoromethyl ester

Prepared from Intermediate 3 using methods similar to that described for Example 1. LCMS retention time 3.38min, *m*/*z* 543 MH⁺

### Example 4: 6α,9α-Difluoro-17α-(1,3-dithiolan-2-ylcarbonyl)oxy-11β-hydroxy-16α-methyl-3-oxo-androsta-1,4-diene-17β-carbothioic acid S-fluoromethyl ester

Prepared from Intermediate 4 using methods similar to that described for Example 1. LCMS retention time 3.60min, *m*/*z* 577 MH⁺

### Example 5: 6α,9α-Difluoro-11β-hydroxy-16α-methyl-17α-(1-methyl-L-prolyl)oxy-3-oxo-androsta-1,4-diene-17β-carbothioic acid S-fluoromethyl ester

Prepared from Intermediate 5 using methods similar to that described for Example 1. LCMS retention time 2.45min, *m*/*z* 556 MH⁺

### Example 6: 6α,9α-Difluoro-11β-hydroxy-16α-methyl-3-oxo-17α-(thietan-3-ylcarbonyl)oxy-androsta-1,4-diene-17β-carbothioic acid S-fluoromethyl ester

Prepared from Intermediate 6 using methods similar to that described for Example 1. LCMS retention time 3.59min, *m*/*z* 545 MH⁺

### Example 7: 6α,9α-Difluoro-11β-hydroxy-16α-methyl-3-oxo-17α-(tetrahydrothiophen-2-ylcarbonyl)oxy-androsta-1,4-diene-17β-carbothioic acid S-fluoromethyl ester

Prepared from Intermediate 7 using methods similar to that described for Example 1. The resulting diasteroisomers were separated by preparative HPLC on a Chiralcel OD column eluting with 20% ethanol in heptane (50ml/min flow rate, room temperature) to give isomer A showing retention time 11.5 min and isomer B showing retention time 17min. Both isomers showed MH⁺ 559 in the mass spectrum.

### Example 8: 6α,9α-Difluoro-11β-hydroxy-16α-methyl-3-oxo-17α-(2-methyltetrahydrofuran-2-ylcarbonyl)oxy-androsta-1,4-diene-17β-carbothioic acid S-fluoromethyl ester

Prepared from Intermediate 8 using methods similar to that described for Example 1. The resulting diasteroisomers were separated by preparative HPLC on a Chiralcel OD column eluting with 10% ethanol in heptane (50ml/min flow rate, room temperature) to give isomer A showing retention time 21 min and isomer B showing retention time 23.5min. Both isomers showed MH⁺ 557 in the mass spectrum.

### Example 9: 6α,9α-Difluoro-17α-(1,3-dioxolan-2-ylcarbonyl)oxy-11β-hydroxy-16α-methyl-3-oxo-androsta-1,4-diene-17β-carbothioic acid S-fluoromethyl ester

Prepared from Intermediate 9 using methods similar to that described for Example 1. LCMS retention time 3.35min, *m*/*z* 545 MH⁺

### Example 10: 6α,9α-Difluoro-11β-hydroxy-16α-methyl-3-oxo-17α-[(2RS,3SR)-3-methyl-tetrahydrofuran-2-ylcarbonyl]oxy-androsta-1,4-diene-17β-carbothioic acid S-fluoromethyl ester

Prepared from Intermediate 10 using methods similar to that described for Example 1. The resulting diasteroisomers were separated by preparative HPLC on a Chiralcel OD column eluting with 10% ethanol in heptane (50ml/min flow rate, room temperature) to give isomer A showing retention time 18 min and isomer B showing retention time 21.5min. Both isomers showed MH⁺ 557 in the mass spectrum.

### Example 11: 6α,9α-Difluoro-11β-hydroxy-16α-methyl-17α-(2-methyl-1,3-dioxoian-2-ylcarbonyl)oxy-3-oxo-androsta-1,4-diene-17β-carbothioic acid S-fluoromethyl ester

Prepared from Intermediate 11 using methods similar to that described for Example 1. LCMS retention time 3.35min, *m*/*z* 559 MH⁺

### Example 12: 6α,9α-Difluoro-11β-hydroxy-16α-methyl-17α-(2-methyl-1,3-dithiolan-2-ylcarbonyl)oxy-3-oxo-androsta-1,4-diene-17β-carbothioic acid S-fluoromethyl ester

Prepared from Intermediate 12 using methods similar to that described for Example 1. LCMS retention time 3.55min, *m*/*z* 591 MH⁺

### Example 13: 6α,9α-Difluoro-11β-hydroxy-16α-methyl-3-oxo-17α-(2-methyl-tetrahydrothiophen-2-ylcarbonyl)oxy-androsta-1,4-diene-17β-carbothioic acid S-fluoromethyl ester

Prepared from Intermediate 13 using methods similar to that described for Example 1.The resulting diasteroisomers were separated by preparative HPLC on a Chiralcel OD column eluting with 10% ethanol in heptane (50ml/min flow rate, room temperature) to give isomer A showing retention time 15.5min and isomer B showing retention time 20.5. Both isomers showed MH⁺ 573 in the mass spectrum.

### Example 14: 6α,9α-Difluoro-11β-hydroxy-16α-methyl-3-oxo-17α-(3-methyltetrahydrofuran-3-ylcarbonyl)oxy-androsta-1,4-diene-17β-carbothioic acid S-fluoromethyl ester

Prepared from Intermediate 14 using methods similar to that described for Example 1. The resulting diasteroisomers were separated by preparative HPLC on a Chiralpak ad column eluting with 15% IPA in heptane (20ml/min flow rate, room temperature) to give isomer A showing retention time 14.5 min and isomer B showing retention time 18min. Both isomers showed MH⁺ 557 in the mass spectrum.

### Example 15: 6α,9α-Difluoro-11β-hydroxy-16α-methyl-3-oxo-17α-(2-ethyl-tetrahydrofuran-2-ylcarbonyl)oxy-androsta-1,4-diene-17β-carbothioic acid S-fluoromethyl ester

Prepared from Intermediate 15 using methods similar to that described for Example 1. The resulting diasteroisomers were separated by preparative HPLC on a Chiralcel OD column eluting with 15% IPA in heptane (20ml/min flow rate, room temperature) to give isomer A showing retention time 19.1 min and isomer B showing retention time 25.5min. Both isomers showed MH⁺ 571 in the mass spectrum.

### Example 16: 6α,9α-Difluoro-17α-(1,3-dithian-2-ylcarbonyl)oxy-11β-hydroxy-16α-methyl-3-oxo-androsta-1,4-diene-17β-carbothioic acid S-fluoromethyl ester

Prepared from Intermediate 16 using methods similar to that described for Example 1. LCMS retention time 3.64min, *m*/*z* 591 MH⁺

### Example 17: 6α,9α-Difluoro-11β-hydroxy-16α-methyl-3-oxo-17α-(tetrahydro-4H-pyran-4-ylcarbonyl)oxy-androsta-1,4-diene-17β-carbothioic acid S-fluoromethyl ester

Prepared from Intermediate 17 using methods similar to that described for Example 1. LCMS retention time 3.42min, *m*/*z* 557 MH⁺

### Preparation of long-acting β₂-adrenoreceptor agonist

### Example X: 3-(4-{[6-({(2R)-2-Hydroxy-2-[4-hydroxy-3-(hydroxymethyl)phenyl]ethyl}amino)-hexyl]oxy}butyl)benzenesulfonamide acetate

i) Di(tert-butyl) 2-(2,2-dimethyl-4H-1,3-benzodioxin-6-yl)-2-oxoethylimidodicarbonate
   Cesium carbonate (70.4g) was added to a stirred suspension of 2-bromo-1-(2,2-dimethyl-4H-1,3-benzodioxin-6-yl)ethanone, (Glaxo, DE 3513885, 1985) (61.8g) and di-t-butyl iminodicarboxylate (47.15g) in acetonitrile (600ml) under nitrogen. After vigorous stirring at 21 °C for 24 h the mixture was diluted with water (*ca*800ml) and the product was extracted with diethyl ether (1 litre, then 200ml). The combined organic layers were washed with brine, dried (MgSO₄) and concentrated to *ca*400ml. The white crystals were collected by filtration, washed with diethyl ether and dried to give the *title compound* (24.4g) δ (CDCl₃) 7.78(1 H, dd, J 8, 2Hz), 7.65 (1 H, brs), 6.87(1 H, d, J 8Hz), 4.97(2H, s), 4.88(2H, s), 1.56(6H, s) and 1.48 (18H, s). Further concentration of the mother liquors gave additional product (13.8g). A third crop (7.1g) was obtained by chromatographing the mother liquors on silica gel, evaporating the appropriate eluate and triturating with diethyl ether.
ii) tert-Butyl 2-(2,2-dimethyl-4H-1,3-benzodioxin-6-yl)-2-oxoethylcarbamate
   Trifluoroacetic acid (92ml) was added to a stirred solution of di(tert-butyl) 2-(2,2-dimethyl-4H-1,3-benzodioxin-6-yl)-2-oxoethylimidodicarbonate, (352.55g) in dichloromethane (3.6litres) at 21 °C and the reaction was stirred for 1.5 h. Aqueous NaOH solution (1.75litres) was added and after 10 min the phases were separated. The organic layer was washed with water, dried (MgSO₄) and evaporated to an oil. This was stored under high vacuum overnight and then triturated with hexane:ether (3:1) to give the crude product (226.61g). This was purified by recrystallisation from diethyl ether to give the *title compound* (122.78g). Further product (61.5g) was obtained from the mother liquors by evaporation and chromatography on a Biotage using 15% ethyl acetate in hexane. LCMS RT = 3.37min.
iii) tert-Butyl (2R)-2-(2,2-dimethyl-4H-1,3-benzodioxin-6-yl)-2-hydroxyethylcarbamate
   A 2M solution of borane - dimethyl sulphide in THF (28ml) was added slowly to a 1M solution of (R)-tetrahydro-1-methyl-3,3-diphenyl-1H,3H-pyrrolo[1,2-c][1,3,2]oxazaborole in toluene (56ml) at 0°C under nitrogen. A solution of tert-butyl 2-(2,2-dimethyl-4H-1,3-benzodioxin-6-yl)-2-oxoethylcarbamate, (108.2g) in THF (1.3litres) was added slowly keeping the temperature below 5°C followed by 2M solution of borane - dimethyl sulphide in THF (252ml) over 50 min. After 1 h, 2M HCl (170ml) was added with cooling and the mixture was partitioned between ethyl acetate and water. The organic layer was washed with saturated NaHCO₃ solution and brine and dried (MgSO₄). The solution was concentrated and the product purified by chromatography on flash silica gel (800g), eluting successively with hexane:ethyl acetate (4:1 then 3:1) to give the *title compound* (93.3g), LCMS RT = 3.31 min.
iv (5R)-5-(2,2-Dimethyl-4H-1,3-benzodioxin-6-yl)-1,3-oxazolidin-2-one
   tert-Butyl (2R)-2-(2,2-dimethyl-4H-1,3-benzodioxin-6-yl)-2-hydroxyethylcarbamate, (86.37g) in DMF (600ml) was added dropwise to a stirred suspension of sodium hydride (60% oil dispersion, 11.9g) in DMF (160ml) with cooling such that the internal temperature remained at 0°C under nitrogen. The mixture was stirred at 21 °C for 2 h. The mixture was recooled to 0°C and 2M HCI (134ml) was added. The mixture was diluted with water and the product was extracted with ethyl acetate twice. The solution was washed with brine twice, dried (MgSO₄) and evaporated to give the *title compound* (63.55g) LCMS RT = 2.66min.
v) 6-Bromohexyl but-3-ynyl ether
   3-Butyn-1-ol (42.4ml) was stirred vigorously with 1,6-dibromohexane (260ml) and tetrabutylammonium bisulphate (2.4g) in 50% aqueous sodium hydroxide solution (200ml) under nitrogen for 3 days. Water (ca 700ml) was added and the organic layer was separated. The aqueous layer was extracted twice with dichloromethane (2 × 100ml) and the combined organic layers were washed with water, dried (MgSO₄) and concentrated. The residue in petroleum ether (bp 40 - 60°) was loaded onto a column of silica gel (1.5kg) and the column was eluted with petroleum ether (bp 40 - 60°C), then 10% diethyl ether in petroleum ether (bp 40 - 60°C) to give the *title compound* (1 03.3g), δ (CDCl₃) 3.56(2H, t, J 7Hz), 3.47(2H, t, J 7Hz), 3.42(2H, t, J 7Hz), 2.45(2H, m), 1.99(1 H, t, J 2Hz), 1.87(2H, m), 1.60(2H, m) and 1.50 to 1.33 (4H, m).
vi) (5R)-3-[6-(But-3-ynyloxy)hexyl]-5-(2,2-dimethyl-4H-1,3-benzodioxin-6-yl)-1,3-oxazolidin-2-one
   (5R)-5-(2,2-dimethyl-4H-1,3-benzodioxin-6-yl)-1,3-oxazolidin-2-one (10g) in DMF (100ml) was added dropwise to a stirred suspension of sodium hydride (60% oil dispersion, 2.33g) in DMF (50ml) with stirring under nitrogen and maintaining the internal temperature at 0°C. Stirring was continued at 0 - 5°C for 1 h. The mixture was recooled to 0°C and a solution of 6-bromohexyl but-3-ynyl ether (14.7g) in DMF (50ml) was added over 1 min. The mixture was then stirred at 20 - 30°C for 2 h. 2M HCI (9ml) was added and the mixture was partitioned between water and diethyl ether. The aqueous layer was extracted with more diethyl ether and the combined organic layers were washed twice with brine. After drying (MgSO₄) the solution was concentrated and loaded onto a column of silica gel (600g) set up in diethyl ether: petroleum ether (bp 40 - 60°C) (1:2). The column was eluted successively with this mixture, then (1:1) and then diethyl ether to give the *title compound* (13.88g) LCMS RT = 3.45min.
vii) 3-[4-({6-[(5R)-5-(2,2-Dimethyl-4H-1,3-benzodioxin-6-yl)-2-oxo-1,3-oxazolidin-3-yl]hexyl}oxy)but-1-ynyl]benzenesulfonamide
   (5R)-3-[6-(But-3-ynyloxy)hexyl]-5-(2,2-dimethyl-4H-1,3-benzodioxin-6-yl)-1,3-oxazolidin-2-one (1.79g) was stirred with 3-iodobenzene sulphonamide (1.4g) in acetonitrile:triethylamine (1:1, 42ml) under nitrogen for 10 min. Cuprous iodide (0.083g) and dichlorobis(triphenylphosphine)palladium (0.192g) were added and the mixture was stirred for 17 h under nitrogen at 21 °C. The mixture was evaporated to dryness and the residue was chromatographed on silica gel (250g) in 30% ethyl acetate: petroleum ether (bp 40 - 60°), then 50%, then 75% and finally ethyl acetate to give the *title compound* (2.35g), LCMS RT = 3.44min.
viii) 3-[4-({6-[(5R)-5-(2,2-Dimethyl-4H-1,3-benzodioxin-6-yl)-2-oxo-1,3-oxazolidin-3-yl]hexyl}oxy)butyl]benzenesulfonamide
   3-[4-({6-[(5R)-5-(2,2-Dimethyl-4H-1,3-benzodioxin-6-yl)-2-oxo-1,3-oxazolidin-3-yl]hexyl}oxy)but-1-ynyl]benzenesulfonamide (2.35g) was stirred with platinum oxide (0.3g) in THF (30ml) under hydrogen for 2 h. The catalyst was removed by filtration using a filter aid and the filter cake was leached with ethyl acetate. The combined filtrates were passed through silica gel (200g) in ethyl acetate and the eluate was evaporated to give the *title compound* (2.32g), LCMS RT = 3.49min.
ix 3-{4-[(6-{[(2R)-2-(2,2-Dimethyl-4H-1,3-benzodioxin-6-yl)-2-hydroxyethyl]amino}hexyl)oxy]butyl}benzenesulfonamide
   3-[4-({6-[(5R)-5-(2,2-Dimethyl-4H-1,3-benzodioxin-6-yl)-2-oxo-1,3-oxazolidin-3-yl]hexyl}oxy)butyl]benzenesulfonamide (0.43g) was stirred in THF (10ml) while purging with a vigorous stream of nitrogen for 5 min. Potassium trimethylsilanoate (0.43g) was added and the mixture was stirred at 70°C under nitrogen for 2.5 h. The mixture was partitioned between dichloromethane and pH 6.4 phosphate buffer and the aqueous layer was extracted with more dichloromethane. The combined organic layers were washed with water, dried (MgSO₄) and concentrated. The residue was purified on silica gel (60g), eluting successively with ethyl acetate:petroleum ether (bp 40 - 60°C) (1:1), ethyl acetate, 10% then 20% methanol in ethyl acetate to give the *title compound* (0.286g), LCMS RT = 2.56min.
x) 3-(4-{[6-({(2R)-2-Hydroxy-2-[4-hydroxy-3-(hydroxymethyl)phenyl]ethyl}amino)-hexyl]oxy}butyl)benzenesulfonamide acetate
   3-{4-[(6-{[(2R)-2-(2,2-dimethyl-4H-1,3-benzodioxin-6-yl)-2-hydroxyethyl]amino}hexyl)oxy]butyl}benzenesulfonamide (0.283g) was stirred with acetic acid (8ml) and water (4ml) at 70° for 35 min before evaporating to dryness. The residue was re-evaporated twice with toluene to give the *title compound* (0.318g) LCMS RT = 2.34min, ES +ve 495 (MH)⁺.

### Pharmacological Activity

Pharmacological activity was assessed in a functional in vitro assay of glucocorticoid agonist activity which is generally predictive of anti-inflammatory or anti-allergic activity in vivo.

The functional assay was based on that described by K.P.Ray et al., Biochem J. (1997), 328, 707-715. A549 cells stably transfected with a reporter gene containing the NF-κB responsive elements from the ELAM gene promoter coupled to sPAP (secreted alkaline phosphatase) were treated with test compounds at appropriate doses for 1 hour at 37°C. The cells were then stimulated with tumour necrosis factor (TNF, 10ng/ml) for 16 hours, at which time the amount of alkaline phosphatase produced is measured by a standard colourimetric assay. Dose response curves were constructed from which EC₅₀ values were estimated.

In this test the compounds of Examples 1 to 17 showed an EC₅₀ value of <1nM.

### Screen for progesterone receptor activity

The human breast cancer cell line T47D has been reported to upregulate an endogenous alkaline phosphatase in response to progestins (Di Lorenzo et al., Cancer Research (1991) 51, 4470-4475. T47D cells were seeded into 96 well plates at a density of 1x10⁵ cells per well and grown overnight at 37°C. Steroids were dissolved in DMSO, added to the cells (final DMSO concentration 0.7%), and incubated for 24 hours at 37°C. The cells were then washed with PBS and lysed with RIPA buffer (1% IGEPAL, 0.5% Na deoxycholate, 0.1% SDS in phosphate buffered saline). Alkaline phosphatase activity was measured spectrophotometrically (405nm) using p-nitrophenylphosphate (1.5mg/ml) as a substrate dissolved in 1 M diethanolamine, 0.28M NaCI, 0.5mM MgCl₂. Dose response curves were constructed from which EC₅₀ values were estimated.

Compounds were tested for progesterone activity in accordance with the above screen and the selectivity was determined by dividing the ED₅₀ at the progesterone receptor by the ED₅₀ at the glucocorticoid receptor.

The selectivity of Example 2 was 246 and Example 4 was 209 (compare fluticasone propionate: selectivity = 18).

In further experiments the following additional data was obtained:

| Compound | | Selectivity |
|---|---|---|
| Fluticasone propionate | | 82 |
| Example 2 | | 1099 |
| Example 4 | | 1192 |
| Example 6 | | 500 |
| Example | 8A | 49425 |
| | 8B | 63855 |
| Example 11 | | 1620 |
| Example 13A | | 2650 |
| | 13B | 1955 |

## Claims

1. A compound of formula (I) 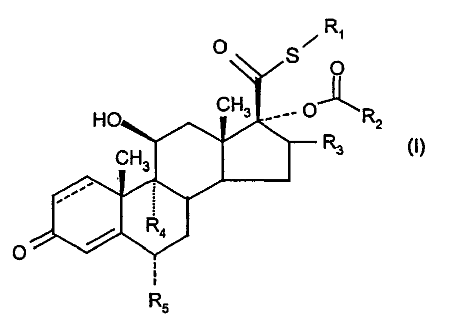 wherein
R₁ represents C₁₋₆ alkyl or C₁₋₆ haloalkyl;
R₂ represents a 4-7 membered non-aromatic ring containing 1-3 heteroatoms selected from O, N and S optionally substituted with one or more methyl, ethyl or halogen groups;
R₃ represents hydrogen, methyl (which may be in either the α or β configuration) or methylene;
R₄ and R₅ are the same or different and each represents hydrogen or halogen; and
-̅-̅-̅-̅-̅ represents a single or a double bond;
and salts and solvates thereof.

2. A compound according to claim 1 in which R₂ represents a 4-7 membered non-aromatic ring containing 1-3 heteroatoms selected from O, N and S optionally substituted with one or more methyl or halogen groups.

3. A compound according to claim 1 or claim 2 in which R₁ represents fluoromethyl, chloromethyl, bromomethyl or 2'-fluoroethyl.

4. A compound according to claim 3 in which R₁ represents fluoromethyl.

5. A compound according to any one of claims 1 to 4 in which R₂ is selected from tetrahydrofuranyl, thietanyl, 1,3-dithiolanyl and pyrrolidinyl, and derivatives substituted by one or more methyl or halogen groups.

6. A compound according to any one of claims 1 to 5 in which R₃ is methyl.

7. A compound according to any of claims 1 to 6 in which R₄ and R₅ are the same or different and each represents hydrogen, fluorine or chlorine.

8. A compound according to any one of claims 1 to 7 in which R₄ and R₅ are the same or different and each represents hydrogen or fluorine.

9. A compound according to any of claims 1 to 8 in which both R₄ and R₅ are fluorine.

10. A compound according to any one of claims 1 to 9 in which -̅-̅-̅-̅-̅ represents a double bond.

11. A compound according to claim 1 which is:
6α,9α-Difluoro-11β-hydroxy-16α-methyl-3-oxo-17α-(tetrahydrofuran-2S-ylcarbonyl)oxy-androsta-1,4-diene-17β-carbothioic acid S-fluoromethyl ester;
6α,9α-Difluoro-11β-hydroxy-16α-methyl-3-oxo-17α-(tetrahydrofuran-2R-ylcarbonyl)oxy-androsta-1,4-diene-17β-carbothioic acid S-fluoromethyl ester;
6α,9α-Difluoro-11β-hydroxy-16α-methyl-3-oxo-17α-(tetrahydrofuran-3-ylcarbonyl)oxy-androsta-1,4-diene-17β-carbothioic acid S-fluoromethyl ester;
6α,9α-Difluoro-17α-(1,3-dithiolan-2-ylcarbonyl)oxy-11β-hydroxy-16α-methyl-3-oxo-androsta-1,4-diene-17β-carbothioic acid S-fluoromethyl ester;
6α,9α-Difluoro-11β-hydroxy-16α-methyl-17α-(1-methyl-L-prolyl)oxy-3-oxo-androsta-1,4-diene-17β-carbothioic acid S-fluoromethyl ester;
6α,9α-Difluoro-11β-hydroxy-16α-methyl-3-oxo-17α-(thietan-3-ylcarbonyl)oxy-androsta-1,4-diene-17β-carbothioic acid S-fluoromethyl ester;
or a salt of solvate of any one thereof.

12. A compound according to claim 1 which is:
6α,9α-Difluoro-11β-hydroxy-16α-methyl-3-oxo-17α-(tetrahydrothiophen-2-ylcarbonyl)oxy-androsta-1,4-diene-17β-carbothioic acid S-fluoromethyl ester;
6α,9α-Difluoro-11β-hydroxy-16α-methyl-3-oxo-17α-(2-methyl-tetrahydrofuran-2R-ylcarbonyl)oxy-androsta-1,4-diene-17β-carbothioic acid S-fluoromethyl ester;
6α,9α-Difluoro-11β-hydroxy-16α-methyl-3-oxo-17α-(2-methyl-tetrahydrofuran-2S-ylcarbonyl)oxy-androsta-1,4-diene-17β-carbothioic acid S-fluoromethyl ester;
6α,9α-Difluoro-17α-(1,3-dioxolan-2-ylcarbonyl)oxy-11β-hydroxy-16α-methyl-3-oxo-androsta-1,4-diene-17β-carbothioic acid S-fluoromethyl ester;
6α,9α-Difluoro-11β-hydroxy-16α-methyl-3-oxo-17α-[(2R,3S)-3-methyltetrahydrofuran-2-ylcarbonyl]oxy-androsta-1,4-diene-17β-carbothioic acid *S-*fluoromethyl ester;
6α,9α-Difluoro-11β-hydroxy-16α-methyl-3-oxo-17α-[(2S,3R)-3-methyltetrahydrofuran-2-ylcarbonyl]oxy-androsta-1,4-diene-17β-carbothioic acid S-fluoromethyl ester;
or a salt or solvate of any one thereof.

13. A compound according to claim 1 which is:
6α,9α-Difluoro-11β-hydroxy-16α-methyl-17α-(2-methyl-1,3-dioxolan-2-ylcarbonyl)oxy-3-oxo-androsta-1,4-diene-17β-carbothioic acid S-fluoromethyl ester;
6α,9α-Difluoro-11β-hydroxy-16α-methyl-17α-(2-methyl-1,3-dithiolan-2-ylcarbonyl)oxy-3-oxo-androsta-1,4-diene-17β-carbothioic acid S-fluoromethyl ester;
6α,9α-Difluoro-11β-hydroxy-16α-methyl-3-oxo-17α-(2-methyl-tetrahydrothiophen-2R-ylcarbonyl)oxy-androsta-1,4-diene-17β-carbothioic acid S-fluoromethyl ester;
6α,9α-Difluoro-11β-hydroxy-16α-methyl-3-oxo-17α-(2-methyl-tetrahydrothiophen-2S-ylcarbonyl)oxy-androsta-1,4-diene-17β-carbothioic acid S-fluoromethyl ester;
6α,9α-Difluoro-11β-hydroxy-16α-methyl-3-oxo-17α-(3-methyltetrahydrofuran-3R-ylcarbonyl)oxy-androsta-1,4-diene-17β-carbothioic acid S-fluoromethyl ester;
6α,9α-Difluoro-11β-hydroxy-16α-methyl-3-oxo-17α-(3-methyltetrahydrofuran-3S-ylcarbonyl)oxy-androsta-1,4-diene-17β-carbothioic acid *S*-fluoromethyl ester;
or a salt or solvate of any one thereof.

14. A compound according to claim 1 which is:
6α,9α-Difluoro-11β-hydroxy-16α-methyl-3-oxo-17α-(2-ethyl-tetrahydrofuran-2R-ylcarbonyl)oxy-androsta-1,4-diene-17β-carbothioic acid *S*-fluoromethyl ester;
6α,9α-Difluoro-11β-hydroxy-16α-methyl-3-oxo-17α-(2-ethyl-tetrahydrofuran-2S-ylcarbonyl)oxy-androsta-1,4-diene-17β-carbothioic acid S-fluoromethyl ester;
6α,9α-Difluoro-17α-(1,3-dithian-2-ylcarbonyl)oxy-11β-hydroxy-16α-methyl-3-oxo-androsta-1,4-diene-17β-carbothioic acid *S*-fluoromethyl ester;
6α,9α-Difluoro-11β-hydroxy-16α-methyl-3-oxo-17α-(tetrahydro-4H-pyran-4-ylcarbonyl)oxy-androsta-1,4-diene-17β-carbothioic acid *S*-fluoromethyl ester
or a salt or solvate of any one thereof.

15. A compound of formula (I) as defined in any of claims 1 to 14 or a physiologically acceptable salt or solvate thereof for use in veterinary or human medicine.

16. Use of a compound of formula (I) as defined in any of claims 1 to 14 or a physiologically acceptable salt or solvate thereof for the manufacture of a medicament for the treatment of inflammatory and/or allergic conditions.

17. A pharmaceutical composition comprising a compound of formula (I) as defined in any of claims 1 to 14 or a physiologically acceptable salt or solvate thereof together, if desirable, in admixture with one or more physiologically acceptable diluents or carriers.

18. A pharmaceutical aerosol formulation comprising a compound of formula (I) as defined in any of claims 1 to 14 or a physiologically acceptable salt or solvate thereof, and a fluorocarbon or hydrogen-containing chlorofluoro carbon as propellant, optionally in combination with a surfactant and or a cosolvent.

19. A pharmaceutical composition according to claim 17 which further comprises another therapeutically active agent.

20. A pharmaceutical composition according to claim 19 in which said another therapeutically active agent is a β₂-adrenoreceptor agonist.

21. A pharmaceutical composition according to claim 20 in which said β₂-adrenoreceptor agonist is a compound of formula (X): 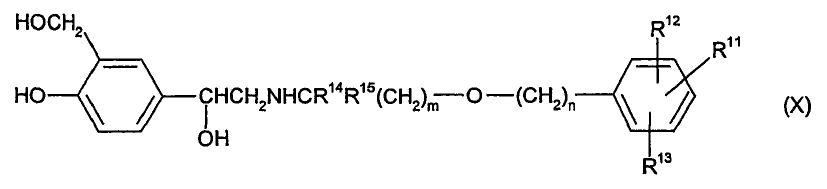 or a salt or solvate thereof, wherein:
m is an integer of from 2 to 8;
n is an integer of from 3 to 11,
with the proviso that m + n is 5 to 19,
R¹¹ is -XSO₂NR¹⁶R¹⁷ wherein X is -(CH₂)ₚ- or C₂₋₆ alkenylene;
R¹⁶ and R¹⁷ are independently selected from hydrogen, C₁₋₆alkyl, C₃₋₇cycloalkyl, C(O)NR¹⁸R¹⁹, phenyl, and phenyl (C₁₋₄alkyl)-,
or R¹⁶ and R¹⁷, together with the nitrogen to which they are bonded, form a 5-, 6-, or 7- membered nitrogen containing ring, and R¹⁶ and R¹⁷ are each optionally substituted by one or two groups selected from halo, C₁₋₆alkyl, C₁₋₆haloalkyl, C₁₋₆alkoxy, hydroxy-substituted C₁₋₆alkoxy, -CO₂R¹⁸, -SO₂NR¹⁸R¹⁹, -CONR¹⁸R¹⁹,-NR¹⁸C(O)R¹⁹, or a 5-, 6- or 7-membered heterocylic ring;
R¹⁸ and R¹⁹ are independently selected from hydrogen, C₁₋₆alkyl, C₃₋₆cycloalkyl, phenyl, and phenyl (C₁₋₄alkyl)-; and
p is an integer of from 0 to 6, preferably from 0 to 4;
R¹² and R¹³ are independently selected from hydrogen, C₁₋₆alkyl, C₁₋₆alkoxy, halo, phenyl, and C₁₋₆haloalkyl; and
R¹⁴ and R¹⁵ are independently selected from hydrogen and C₁₋₄alkyl with the proviso that the total number of carbon atoms in R¹⁴ and R¹⁵ is not more than 4.

22. A pharmaceutical composition according to claim 21 in which the compound of formula (X) is 3-(4-{[6-({(2R)-2-Hydroxy-2-[4-hydroxy-3-(hydroxymethyl)phenyl]ethyl}amino)-hexyl]oxy}butyl)benzenesulfonamide or a salt or solvate thereof.

23. A process for preparing a compound of formula (I) according to claim 1 which comprises alkylation of a compound of formula (II) 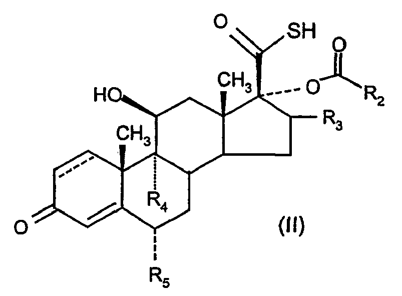 wherein R₂, R₃, R₄, R₅ and -̅-̅-̅-̅-̅ are as defined in claim 1.

24. A process according to claim 23 wherein alkylation is performed by reacting the compound of formula (II) with an appropriate alkyl or haloalkyl halide.

25. A compound of formula (II) 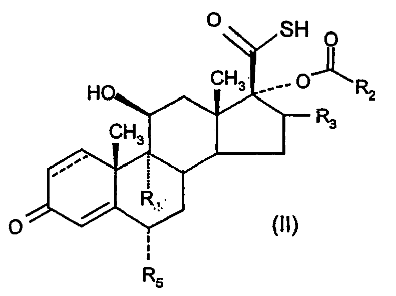 wherein R₂, R₃, R₄, R₅ and -̅-̅-̅-̅-̅ are as defined in claim 1.

## Patentansprüche

1. Verbindung der Formel (I): 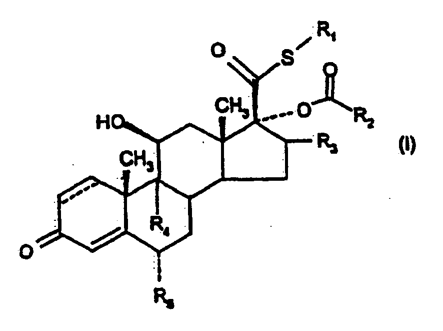 worin.
R₁ C₁₋₆-Alkyl oder C₁₋₆-Halogenalkyl repräsentiert;
R₂ repräsentiert einen 4- bis 7-gliedrigen nicht-aromatischen Ring, enthaltend 1 bis 3 Heteroatome, gewählt aus 0, N und S, optional substituiert mit ein oder mehr Methyl-, Ethyl- oder Halogen-Gruppen;
R₃ repräsentiert Wasserstoff, Methyl (das sich entweder in der α- oder β-Konfiguration befinden kann) oder Methylen;
R₄ und R₅ sind dieselben oder unterschiedlich und bedeuten jeweils Wasserstoff oder Halogen und
-̅-̅-̅-̅-̅ repräsentiert eine Einzel- oder eine Doppelbindung;
und Salze und Solvate davon.

2. Verbindung gemäß Anspruch 1, worin R₂ einen 4- bis 7-gliedrigen nicht-aromatischen Ring repräsentiert, enthaltend 1 bis 3 Heteroatome, gewählt aus 0, N und S, optional substituiert mit ein oder mehr Methyl- oder Halogen-Gruppen.

3. Verbindung gemäß Anspruch 1 oder 2, worin R₁ Fluormethyl, Chlormethyl, Brommethyl oder 2'-Fluorethyl repräsentiert.

4. Verbindung gemäß Anspruch 3, worin R₁ Fluormethyl repräsentiert.

5. Verbindung gemäß einem der Ansprüche 1 bis 4, worin R₂ gewählt ist aus Tetrahydrofuranyl, Thietanyl, 1,3-Dithiolanyl und Pyrrolidinyl und Derivate, substituiert durch ein oder mehr Methyl- oder Halogen-Gruppen.

6. Verbindung gemäß einem der Ansprüche 1 bis 5, worin R₃ Methyl ist.

7. Verbindung gemäß einem der Ansprüche 1 bis 6, worin R₄ und R₅ dieselben oder unterschiedlich sind und jeweils Wasserstoff, Fluor oder Chlor repräsentieren.

8. Verbindung gemäß einem der Ansprüche 1 bis 7, worin R₄ und R₅ dieselben oder unterschiedlich sind und jeweils Wasserstoff oder Fluor repräsentieren.

9. Verbindung gemäß einem der Ansprüche 1 bis 8, worin beide R₄ und R₅ Fluor sind.

10. Verbindung gemäß einem der Ansprüche 1 bis 9, worin eine Doppelbindung repräsentiert.

11. Verbindung gemäß Anspruch 1, die folgendes ist:
6α,9α-Difluor-11β-hydroxy-16α-methyl-3-oxo-17α-(tetrahydrofuran-2S-ylcarbonyl)oxy-androsta-1,4-dien-17β-thiocarbonsäure-S-fluormethylester;
6α,9α-Difluor-11β-hydroxy-16α-methyl-3-oxo-17α-(tetrahydrofuran-2R-ylcarbonyl)oxy-androsta-1,4-dien-17β-thiocarbonsäure-S-fluormethylester;
6α,9α-Difluor-11β-hydroxy-16α-methyl-3-oxo-17α-(tetrahydrofuran-3-ylcarbonyl)oxy-androsta-1,4-dien-17β-thiocarbonsäure-S-fluormethylester;
6α,9α-Difluor-17α-(2,3-dithiolan-2-ylcarbonyl)oxy-11β-hydroxy-16α-methyl-3-oxo-androsta-1,4-dien-17β-thiocarbonsäure-S-fluormethylester;
6α,9α-Difluor-11β-hydroxy-16α-methyl-17α-(1-methyl-L-prolyl)oxy-3-oxo-androsta-1,4-dien-17β-thiocarbonsäure-S-fluormethylester;
6α,9α-Difluor-11β-hydroxy-16α-methyl-3-oxo-17α-(thietan-3-ylcarbonyl)oxy-androsta-1,4-dien-17β-thiocarbonsäure-S-fluormethylester;
oder ein Salz oder Solvat von irgendeiner davon.

12. Verbindung gemäß Anspruch 1, die folgendes ist:
6α,9α-Difluor-11β-hydroxy-16α-methyl-3-oxo-17α-(tetrahydrothiophen-2-ylcarbonyl)oxy-androsta-1,4-dien-17β-thiocarbonsäure-S-fluormethylester;
6α,9α-Difluor-11β-hydroxy-16α-methyl-3-oxo-17α-(2-methyl-tetrahydrofuran-2R-ylcarbonyl)oxy-androsta-1,4-dien-17β-thiocarbonsäure-S-fluormethylester;
6α,9α-Difluor-11β-hydroxy-16α-methyl-3-oxo-17α-(2-methyl-tetrahydrofuran-2S-ylcarbonyl)oxy-androsta-1,4-dien-17β-thiocarbonsäure-S-fluormethylester;
6α,9α-Difluor-17α-(1,3-dioxolan-2-ylcarbonyl)oxy-11β-hydroxy-16α-methyl-3-oxo-androsta-1,4-dien-17β-thiocarbonsäure-S-fluormethylester;
6α,9α-Difluor-11β-hydroxy-16α-methyl-3-oxo-17α[(2R,3S)-3-methyl-tetrahydrofuran-2-ylcarbonyl]oxy-androsta-1,4-dien-17β-thiocarbonsäure-S-fluormethylester;
6α,9α-Difluor-11β-hydroxy-16α-methyl-3-oxo-17α[(2S,3R)-3-methyl-tetrahydrofuran-2-ylcarbonyl]oxy-androsta-1,4-dien-17β-thiocarbonsäure-S-fluormethylester;
oder ein Salz oder Solvat von irgendeiner davon.

13. Verbindung gemäß Anspruch 1, die folgendes ist:
6α,9α-Difluor-11β-hydroxy-16α-methyl-17α-(2-methyl-1,3-dioxolan-2-ylcarbonyl)oxy-3-oxo-androsta-1,4-dien-17β-thiocarbonsäure-S-fluormethylester;
6α,9α-Difluor-11β-hydroxy-16α-methyl-17α-(2-methyl-1,3-dithiolan-2-ylcarbonyl)oxy-3-oxo-androsta-1,4-dien-17β-thiocarbonsäure-S-fluormethylester;
6α,9α-Difluor-11β-hydroxy-16α-methyl-3-oxo-17α-(2-methyl-tetrahydrothiophen-2R-ylcarbonyl)oxy-androsta-1,4-dien-17β-thiocarbonsäure-S-fluormethylester;
6α,9α-Difluor-11β-hydroxy-16α-methyl-3-oxo-17α-(2-methyl-tetrahydrothiophen-2S-ylcarbonyl)oxy-androsta-1,4-dien-17β-thiocarbonsäure-S-fluormethylester;
6α,9α-Difluor-11β-hydroxy-16α-methyl-3-oxo-17α-(3-methyltetrahydrofuran-3R-ylcarbonyl)oxy-androsta-1,4-dien-17β-thiocarbonsäure-S-fluormethylester;
6α,9α-Difluor-11β-hydroxy-16α-methyl-3-oxo-17α-(3-methyltetrahydrofuran-3S-ylcarbonyl)oxy-androsta-1,4-dien-17β-thiocarbonsäure-S-fluormethylester;
oder ein Salz oder Solvat von irgendeiner davon.

14. Verbindung gemäß Anspruch 1, die folgendes ist:
6α,9α-Difluor-11β-hydroxy-16α-methyl-3-oxo-17α-(2-ethyl-tetrahydrofuran-2R-ylcarbonyl)oxy-androsta-1,4-dien-17β-thiocarbonsäure-S-fluormethylester;
6α,9α-Difluor-11β-hydroxy-16α-methyl-3-oxo-17α-(2-ethyl-tetrahydrofuran-2S-ylcarbonyl)oxy-androsta-1,4-dien-17β-thiocarbonsäure-S-fluormethylester;
6α,9α-Difluor-17α-(1,3-dithian-2-ylcarbonyl)oxy-11β-hydroxy-16α-methyl-3-oxo-androsta-1,4-dien-17β-thiocarbonsäure-S-fluormethylester;
6α,9α-Difluor-11β-hydroxy-16α-methyl-3-oxo-17α-(tetrahydro-4H-pyran-4-ylcarbonyl)oxy-androsta-1,4-dien-17β-thiocarbonsäure-S-fluormethylester;
oder ein Salz oder Solvat von irgendeiner davon.

15. Verbindung gemäß Formel (I) wie definiert in einem der Ansprüche 1 bis 14 oder ein physiologisch annehmbares Salz oder Solvat davon zur Verwendung in der Veterinärmedizin oder Humanmedizin.

16. Verwendung einer Verbindung der Formel (I) wie definiert in irgendeinem der Ansprüche 1 bis 14 oder eines physiologisch annehmbaren Salzes oder Solvats davon für die Herstellung eines Medikaments zur Behandlung entzündlicher und/oder allergischer Zustände.

17. Pharmazeutische Zusammensetzung, umfassend eine Verbindung der Formel (I) wie definiert in einem der Ansprüche 1 bis 14 oder ein physiologisch annehmbares Salz oder Solvat davon, zusammen falls gewünscht, in Mischung mit ein oder mehr physiologisch annehmbaren Verdünnungsmitteln oder Trägern.

18. Pharmazeutische Aerosol-Formulierung, umfassend eine Verbindung gemäß Formel (I) wie definiert in einem der Ansprüche 1 bis 14 oder ein physiologisch annehmbares Salz oder Solvat davon und einen Fluorkohlenstoff- oder Wasserstoff-haltiger Chlorfluorkohlenstoff als Treibmittel, optional in Kombination mit einem Tensid oder einem Co-Lösungsmittel.

19. Pharmazeutische Zusammensetzung gemäß Anspruch 17, die weiterhin ein anderes therapeutisch wirksames Mittel umfaßt.

20. Pharmazeutische Zusammensetzung gemäß Anspruch 19, worin das andere therapeutisch aktive Mittel ein β₂-Adrenorezeptor-Agonist ist.

21. Pharmazeutische Zusammensetzung gemäß Anspruch 20, worin der β₂-Adrenorezeptor-Agonist eine Verbindung der Formel (X) ist: 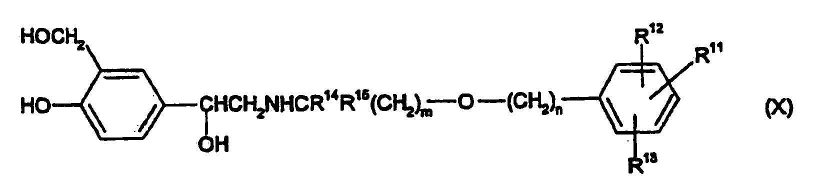 oder ein Salz oder Solvat davon, worin:
m eine ganze Zahl von 2 bis 8 ist;
n ist eine ganze Zahl von 3 bis 11,
mit der Maßgabe, daß m + n 5 bis 19 ist,
R¹¹ ist -XSO₂NR¹⁶R¹⁷, worin X -(CH₂)ₚ- oder C₂₋₆-Alkenylen ist;
R¹⁶ und R¹⁷ werden unabhängig gewählt aus Wasserstoff, C₁₋₆-Alkyl, C₃₋₇-Cycloalkyl, C(O)NR¹⁸R¹⁹, Phenyl und
Phenyl(C₁₋₄-alkyl)- oder R¹⁶ und R¹⁷ bilden zusammen mit dem Stickstoff, an den sie gebunden sind, einen 5-, 6- oder 7-gliedrigen stickstoffhaltigen Ring und R¹⁶ und R¹⁷ sind jeweils optional substituiert durch ein oder zwei Gruppen, gewählt aus Halogen, C₁₋₆-Alkyl, C₁₋₆-Halogenalkyl, C₁₋₆-Alkoxy, Hydroxy-substituiertem C₁₋₆-Alkoxy, -CO₂R¹⁸, -SO₂NR¹⁸R¹⁹, -CONR¹⁸R¹⁹, -NR¹⁸C(O)R¹⁹ oder einem 5-, 6- oder 7-gliedrigen heterocyclischen Ring;
R¹⁸ und R¹⁹ werden unabhängig gewählt aus Wasserstoff, C₁₋₆-Alkyl, C₃₋₆-Cycloalkyl, Phenyl und Phenyl-(C₁₋₄-alkyl)-; und
p ist eine ganze Zahl von 0 bis 6, vorzugsweise von 0 bis 4;
R¹² und R¹³ sind unabhängig gewählt aus Wasserstoff, C₁₋₆-Alkyl, C₁₋₆-Alkoxy, Halogen, Phenyl und C₁₋₆-Halogenalkyl; und
R¹⁴ und R¹⁵ sind unabhängig gewählt aus Wasserstoff und C₁₋₄-Alkyl, mit der Maßgabe, daß die Gesamtzahl an Kohlenstoffatomen in R¹⁴ und R¹⁵ nicht mehr als 4 ist.

22. Pharmazeutische Zusammensetzung gemäß Anspruch 21, worin die Verbindung der Formel (X) 3-(4-{[6-({(2R)-2-Hydroxy-2-[4-hydroxy-3-(hydroxymethyl)-phenyl]ethyl}amino)-hexyl]oxy}butyl)benzolsulfonamid oder ein Salz oder Solvat davon ist.

23. Verfahren zur Herstellung einer Verbindung der Formel (I) gemäß Anspruch 1, das die Alkylierung einer Verbindung der Formel (II) umfaßt: 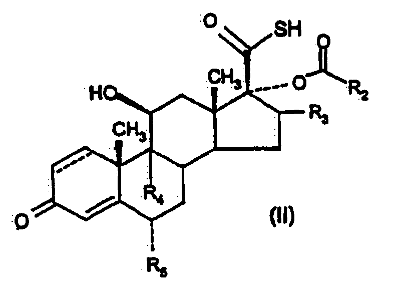 worin R₂, R₃, R₄, R₅ und
-̅-̅-̅-̅-̅ wie in Anspruch 1 definiert sind.

24. Verfahren gemäß Anspruch 23, worin die Alkylierung durch Umsetzung der Verbindung der Formel (II) mit einem geeigneten Alkyl oder Halogenalkylhalogenid durchgeführt wird.

25. Verbindung der Formel (II) 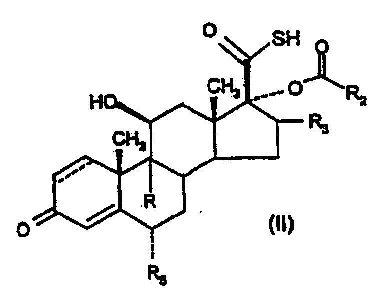 worin R₂, R₃, R₄, R₅ und
-̅-̅-̅-̅-̅ wie in Anspruch 1 definiert sind.

## Revendications

1. Composé de formule (I) : 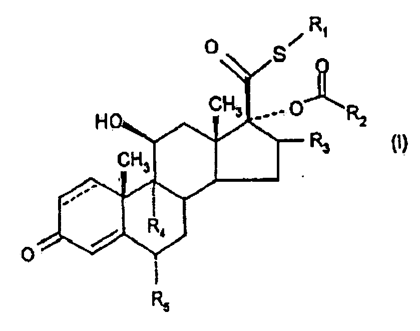 dans laquelle
- R₁ représente un groupe alkyle C₁₋₆ ou haloalkyle C₁₋₆;
- R₂ représente un cycle non aromatique de 4 à 7 atomes contenant 1 à 3 hétéroatomes choisis parmi O, N et S, éventuellement substitué par un ou plusieurs groupes méthyle, éthyle ou halogène ;
- R₃ représente l'hydrogène, un groupe méthyle (qui peut être en configuration soit α, soit β) ou méthylène ;
- R₄ et R₅ sont identiques ou différents et représentent chacun l'hydrogène ou un halogène, et -̅-̅-̅-̅-̅ représente une liaison simple ou une double liaison ;
et sels et solvates de celui-ci.

2. Composé selon la revendication 1, dans lequel R₂ représente un cycle non aromatique de 4 à 7 atomes contenant 1 à 3 hétéroatomes choisis parmi O, N et S, éventuellement substitué par un ou plusieurs groupes méthyle ou halogène.

3. Composé selon la revendication 1 ou la revendication 2, dans lequel R₁ représente un groupe fluorométhyle, chlorométhyle, bromométhyle ou 2'-fluoroéthyle.

4. Composé selon la revendication 3, dans lequel R₁ représente le groupe fluorométhyle.

5. Composé selon l'une quelconque des revendications 1 à 4, dans lequel R₂ est choisi parmi les groupes tétrahydrofuranyle, thiétanyle, 1,3-dithiolanyle et pyrrolidinyle, et dérivés substitués par un ou plusieurs groupes méthyle ou halogène.

6. Composé selon l'une quelconque des revendications 1 à 5, dans lequel R₂ représente un groupe méthyle.

7. Composé selon l'une quelconque des revendications 1 à 6, dans lequel R₄ et R₅ sont identiques ou différents et représentent chacun l'hydrogène, le fluor ou le chlore.

8. Composé selon l'une quelconque des revendications 1 à 7, dans lequel R₄ et R₅ sont identiques ou différents et représentent chacun l'hydrogène ou le fluor.

9. Composé selon l'une quelconque des revendications 1 à 8, dans lequel R₄ et R₅ représentent tous deux le fluor.

10. Composé selon l'une quelconque des revendications 1 à 9, dans lequel -̅-̅-̅-̅-̅ représente une double liaison.

11. Composé selon la revendication 1, qui est :
- l'ester S-fluorométhyle de l'acide 6α,9α-difluoro-11β-hydroxy-16α-méthyl-3-oxo-17α-(tétrahydrofuran-2S-ylcarbonyl)oxy-androsta-1,4-diène-17β-carbothioïque ;
- l'ester S-fluorométhyle de l'acide 6α,9α-difluoro-11β-hydroxy-16α-méthyl-3-oxo-17α-(tétrahydrofuran-2R-ylcarbonyl)oxy-androsta-1,4-diène-17β-carbothioïque ;
- l'ester S-fluorométhyle de l'acide 6α,9α-difluoro-11β-hydroxy-16α-méthyl-3-oxo-17α-(tétrahydrofuran-3-ylcarbonyl)oxy-androsta-1,4-diène-17β-carbothioïque ;
- l'ester S-fluorométhyle de l'acide 6α,9α-difluoro-17α-(1,3-dithiolan-2-ylcarbonyl)oxy-11β-hydroxy-16α-méthyl-3-oxo-androsta-1,4-diène-17β-carbothioïque ;
- l'ester S-fluorométhyle de l'acide 6α,9α-difluoro-11β-hydroxy-16α-méthyl-17a-( 1-méthyl-L-prolyl)oxy-3-oxo-androsta-1,4-diène-17β-carbothioïque ;
- l'ester S-fluorométhyle de l'acide 6α,9α-difluoro-11β-hydroxy-16α-méthyl-3-oxo-17α-(thiétan-3-ylcarbonyl)oxy-androsta-1,4-diène-17β-carbothioïque ;
ou un sel ou solvate de ceux-ci.

12. Composé selon la revendication 1, qui est :
- l'ester S-fluorométhyle de l'acide 6α,9α-difluoro-11β-hydroxy-16α-méthyl-3-oxo-17α-(tétrahydrothiophén-2-ylcarbonyl)oxy-androsta-1,4-diène-17β-carbothioïque ;
- l'ester S-fluorométhyle de l'acide 6α,9α-difluoro-11β-hydroxy-16α-méthyl-3-oxo-17α-(2-méthyl-tétrahydrofuran-2R-ylcarbonyl)oxy-androsta-1,4-diène-17β-carbothioïque ;
- l'ester S-fluorométhyle de l'acide 6α,9α-difluoro-11β-hydroxy-16α-méthyl-3-oxo-17α-(2-méthyl-tétrahydrofuran-2S-ylcarbonyl)oxy-androsta-1,4-diène-17β-carbothioïque ;
- l'ester S-fluorométhyle de l'acide 6α,9α-difluoro-17α-(1,3-dioxolan-2-ylcarbonyl)oxy-11 β-hydroxy-16α-méthyl-3-oxo-androsta-1,4-diène-17β-carbothioïque ;
- l'ester S-fluorométhyle de l'acide 6α,9α-difluoro-11β-hydroxy-16α-méthyl-3-oxo-17α-[(2R,3S)-3-méthyl-tétrahydrofuran-2-ylcarbonyl]oxy-androsta-1,4-diène-17β-carbothioïque ;
- l'ester S-fluorométhyle de l'acide 6α,9α-difluoro-11β-hydroxy-16α-méthyl-3-oxo-17α-[(2S,3R)-3-méthyl-tétrahydrofuran-2-ylcarbonyl]oxy-androsta-1,4-diène-17β-carbothioïque ;
ou un sel ou solvate de ceux-ci.

13. Composé selon la revendication 1, qui est :
- l'ester S-fluorométhyle de l'acide 6α,9α-difluoro-11β-hydroxy-16α-méthyl-17α-(2-méthyl-1,3-dioxolan-2-ylcarbonyl)oxy-3-oxo-androsta-1,4-diène-17β-carbothioïque ;
- l'ester S-fluorométhyle de l'acide 6α,9α-difluoro-11β-hydroxy-16α-méthyl-17α-(2-méthyl-1,3-dithiolan-2-ylcarbonyl)oxy-3-oxo-androsta-1,4-diène-17β-carbothioïque ;
- l'ester S-fluorométhyle de l'acide 6α,9α-difluoro-11β-hydroxy-16α-méthyl-3-oxo-17α-(2-méthyl-tétrahydrothiophén-2R-ylcarbonyl)oxy-androsta-1,4-diène-17β-carbothioïque ;
- l'ester S-fluorométhyle de l'acide 6α,9α-difluoro-11β-hydroxy-16α-méthyl-3-oxo-17α-(2-méthyl-tétrahydrothiophén-2S-ylcarbonyl)oxy-androsta-1,4-diène-17β-carbothioïque ;
- l'ester S-fluorométhyle de l'acide 6α,9α-difluoro-11β-hydroxy-16α-méthyl-3-oxo-17α-(3-méthyltétrahydrofuran-3R-ylcarbonyl)oxy-androsta-1,4-diène-17β-carbothioïque ;
- l'ester S-fluorométhyle de l'acide 6α,9α-difluoro-11β-hydroxy-16α-méthyl-3-oxo-17α-(3-méthyltétrahydrofuran-3S-ylcarbonyl)oxy-androsta-1,4-diène-17β-carbothioïque ;
ou un sel ou solvate de ceux-ci.

14. Composé selon la revendication 1, qui est :
- l'ester S-fluorométhyle de l'acide 6α,9α-difluoro-11β-hydroxy-16α-méthyl-3-oxo-17α-(2-éthyl-tétrahydrofuran-2R-ylcarbonyl)oxy-androsta-1,4-diène-17β-carbothioïque
- l'ester S-fluorométhyle de l'acide 6α,9α-difluoro-11β-hydroxy-16α-méthyl-3-oxo-17a-(2-éthyl-tétrahydrofuran-2S-ylcarbonyl)oxy-androsta-1,4-diène-17β-carbothioïque ;
- l'ester S-fluorométhyle de l'acide 6α,9α-difluoro-17α-(1,3-dithian-2-ylcarbonyl)oxy-11β-hydroxy-16α-méthyl-3-oxo-androsta-1,4-diène-17β-carbothioïque ;
- l'ester S-fluorométhyle de l'acide 6α,9α-difluoro-11β-hydroxy-16α-méthyl-3-oxo-17α-(tétrahydro-4H-pyran-4-ylcarbonyl)oxy-androsta-1,4-diène-17β-carbothioïque ;
ou un sel ou solvate de ceux-ci.

15. Composé de formule (I) selon l'une quelconque des revendications 1 à 14, ou un de ses sels ou solvates physiologiquement acceptables, pour l'utilisation en médecine vétérinaire ou humaine.

16. Utilisation d'un composé de formule (I) selon l'une quelconque des revendications 1 à 14, ou d'un de ses sels ou solvates physiologiquement acceptables, dans la fabrication d'un médicament destiné au traitement d'états inflammatoires et/ou allergiques.

17. Composition pharmaceutique comprenant un composé de formule (I) selon l'une quelconque des revendications 1 à 14, ou un de ses sels ou solvates physiologiquement acceptables, associé si cela est souhaitable, sous forme de mélange avec un ou plusieurs diluants ou véhicules physiologiquement acceptables.

18. Formulation pharmaceutique aérosol comprenant un composé de formule (I) selon l'une quelconque des revendications 1 à 14, ou un de ses sels ou solvates physiologiquement acceptables, et en tant que propulseur, un fluorocarbure ou un chlorofluorocarbure contenant de l'hydrogène, éventuellement en combinaison avec un tensio-actif ou un co-solvant.

19. Composition pharmaceutique selon la revendication 17, qui comprend aussi un autre agent thérapeutiquement actif.

20. Composition pharmaceutique selon la revendication 19, dans laquelle ledit autre agent thérapeutiquement actif est un agoniste adrénorécepteurs-β₂.

21. Composition pharmaceutique selon la revendication 20, dans laquelle ledit agoniste adrénorécepteurs-β₂ est un composé de formule (X) : 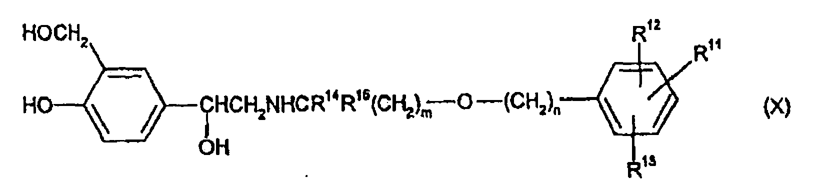 ou un de ses sels ou solvates,
formule dans laquelle :
- m est un nombre entier de 2 à 8 ;
- n est un nombre entier de 3 à 11 ;
à la condition que m+n soit de 5 à 19,
- R¹¹ représente un groupe -XSO₂NR¹⁶R¹⁷ dans lequel X représente un groupe -(CH₂)ₚ- ou alkénylène C₂₋₆;
- R¹⁶ et R¹⁷ sont choisis indépendamment parmi l'hydrogène et les groupes alkyle C₁₋₆, cycloalkyle C₃₋₇, C(O)NR¹⁸R¹⁹, phényle et phénylalkyle C₁₋₄;
ou bien R¹⁶ et R¹⁷, associés à l'azote auquel ils sont liés, forment un cycle de 5, 6 ou 7 atomes contenant de l'azote, et R¹⁶ et R¹⁷ sont chacun éventuellement substitués par un ou deux groupes choisis parmi les groupes halo, alkyle C₁₋₆, haloalkyle C₁₋₆, alcoxy C₁₋₆, alcoxy C₁₋₆ à substituant hydroxy, -CO₂R¹⁸, -SO₂NR¹⁸R¹⁹, -CONR¹⁸R¹⁹, -NR¹⁸C(O)R¹⁹
ou un cycle hétérocyclique à 5, 6 ou 7 atomes ;
- R¹⁸ et R¹⁹ sont choisis indépendamment parmi l'hydrogène, les groupes alkyle C₁₋₆, cycloalkyle C₃₋₆, phényle et phénylalkyle C₁₋₄; et
- p est un nombre entier de 0 à 6, de préférence de 0 à 4 ;
- R¹² et R¹³ sont choisis indépendamment parmi l'hydrogène, les groupes alkyle C₁₋₆, alcoxy C₁₋₆, halo, phényle et haloalkyle C₁₋₆; et
- R¹⁴ et R¹⁵ sont choisis indépendamment parmi l'hydrogène et les groupes alkyle C₁₋₄, à la condition que le nombre total d'atomes de carbone dans R¹⁴ et R¹⁵ ne soit pas supérieur à 4.

22. Composition pharmaceutique selon la revendication 21, dans laquelle le composé de formule (X) est le 3-(4-{[6-({(2R)-2-hydroxy-2-[4-hydroxy-3-(hydroxyméthyl)phényl]éthyl}amino)hexyl]-oxy}butyl)benzènesulfonamide ou un de ses sels ou solvates.

23. Procédé de préparation d'un composé de formule (I) selon la revendication 1, qui comprend l'alkylation d'un composé de formule (II) : 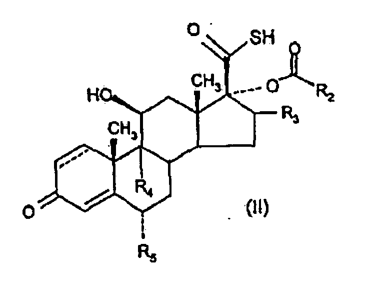 dans laquelle R₂, R₃, R₄, R₅ et -̅-̅-̅-̅_{.} sont tels que définis dans la revendication 1.

24. Procédé selon la revendication 23, dans lequel l'alkylation est effectuée par la réaction du composé de formule (II) avec un halogénure d'alkyle ou d'haloalkyle approprié.

25. Composé de formule (II) 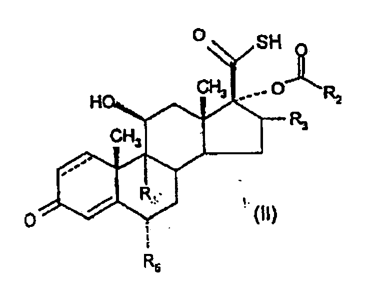 dans laquelle R₂, R₃, R₄, R₅ et -̅-̅-̅-̅.̅ sont tels que définis dans la revendication 1.
